# EUROPEAN PATENT APPLICATION

(11) **EP 4 592 391 A2**
(43) Date of publication of application: **30.07.2025**
(21) Application number: 25171501.7
(22) Date of filing: 21.10.2016
(51) Int. Cl.: C12N 15/113

(54) **METHODS AND COMPOSITIONS FOR MODIFYING MACROPHAGE POLARIZATION INTO PRO-INFLAMMATORY CELLS TO TREAT CANCER**

(30) Priority: 21.10.2015 EP 15190918
(62) Divisional of application: 23151596.6
(71) Applicant: OSE IMMUNOTHERAPEUTICS, 44200 Nantes (FR)
(72) Inventor: POIRIER, Nicolas, 44119 Grandchamps Des Fontaines (FR); VANHOVE, Bernard, 44400 Reze (FR); GAUTTIER, Vanessa, 44400 Reze (FR)
(74) Representative: Ernest Gutmann - Yves Plasseraud S.A.S.

(57) **Abstract**

The present invention concerns the use of an anti-SIRPa compound in combination with a second therapeutic agent selected from the group consisting of a blocking anti-CTLA-4 antibody and an agonistic anti-CD137 antibody. Such a combination is used to treat cancer. Interestingly, this invention allows to treat cancer through an indirect pathway involving the immune system.

## Description

### FIELD OF THE INVENTION

The present invention pertains to the field of immunotherapy. More specifically, the present invention provides a method for inhibiting M2-type macrophages polarization in order to induce a pro-inflammatory environment and consequently allows appropriate immune responses in cancers, infectious diseases, vaccination, trauma and chronic inflammatory diseases.

The present invention concerns in particular the use of an anti-SIRPa compound able to inhibit the polarization of anti-inflammatory M2-type macrophages and/or favors pro-inflammatory M1-type macrophages. In a preferred embodiment, such compound is used to treat cancer. Interestingly, this invention allows to treat cancer through an indirect pathway involving the immune system.

### BACKGROUND AND PRIOR ART

Cancers resulting from uncontrolled cell proliferation form a group of varied diseases. Surgery and radiation therapy do not treat all cancers, especially metastatic stages. More effective treatments are supposed to reach every organ of the patient: it is the case with modern chemotherapy that can induce the death of tumor cells. However, the cytotoxic effect of the drugs remains a major obstacle to chemotherapy.

The rise of molecular biology and genetics has allowed the understanding of the mechanisms leading to cancer cell development and of "targeted therapies". These treatments, combined with chemotherapy, specifically attack tumor cells, sparing healthy cells. However, although these therapies significantly lengthen life expectancy of patients, none has yet resulted in healing. Today, it is well established that human tumor cells may be resistant to treatment and escape to the surveillance by the immune system. Combination therapies are thus essential to cure a patient, but with the drawback of multiplying the problems of side effects.

Initial research conducted in the field of cancer immunotherapy aimed at "boosting" the effector cells of the immune system, making them more aggressive towards tumors. This strategy has in fact proved somewhat successful.

New generation immunotherapeutic molecules, revolutionizing cancer treatments, block suppressor mechanisms of these cells, allowing the effector T cells (Teff) to exercise their action. This is the concept called "Inhibit inhibitors". An anti-CTLA-4 antibody (Yervoy^{®}) has been the first molecule for treating metastatic forms of malignant melanoma prolonging the mean survival of patients of 6 to 10 months, with a quarter of patients still alive after 2 years. Unfortunately, these results, as spectacular as they are, still do not cure most patients.

The present invention relies on an "inhibit inhibitors" approach and provides a new method useful in immunotherapy. More specifically, the present invention pertains to a method for modifying macrophage polarization in order to induce a pro-inflammatory environment. The method consists in the use of an anti-SIRPa compound able to inhibit the polarization of anti-inflammatory M2-type macrophages and/or favors pro-inflammatory M1-type macrophages, for inhibiting the anti-inflammatory signal provided by M2-type macrophages and favoring the pro-inflammatory signal provided by M1-type macrophages. This approach allows to reestablish an inflammatory environment favorable to the action of the T effector cells, in particular in eliminating the cancer cells.

### Macrophage plasticity and polarization

Macrophages are cells that have the highest plasticity of the hematopoietic system. They are involved both in innate immunity (phagocytosis capacity) and in adaptive immunity (cell polarization), but also in ontogeny, in homeostasis and in tissue repair (Mantovani et al., 2013; Wynn et al., 2013). Macrophages are present in all tissues. They have a large phenotypic and functional diversity. During ontogeny, these cells also exhibit a diversity of origins which persists into adulthood. In the tissues, monocytes-macrophages respond to environmental stimuli (product from microbial infection, damaged cells, activated lymphocytes) and acquire distinct phenotypes. For a long time, these cells have been classified according to their function in a binary manner in connection with the inflammatory condition.

Depending on stimuli monocyte-macrophage received, they reprogram their transcriptome, resulting in distinct functional and phenotypic spectra. Macrophages are categorized simplistically into 2 sub-populations or states of polarization (or activation): classical activation phenotype M1 and the alternative activation phenotype M2 (Gabrilovich et al., 2012). The M1 classification is associated *in vitro* with the use of IFNg factor alone or in combination with microbial factors such as LPS or inflammatory cytokines such as TNF-α and GM-CSF. The polarization M2 is rather associated with the IL4 or IL13 (Stein et al., 1992). Other cytokines are also identified as inducing M2 type polarization such as IL33, which induces overexpression of Arg1 (arginase 1), CCL24 or CCL17 playing a role in inflammation. The IL21 and more commonly CSF1 are major players in the polarization of macrophages. Macrophages may also acquire the status of "M2-like", sharing common characteristics of M2. In fact, a large number of stimuli such as immune complexes associated with LPS, IL-1, glucocorticoids, TGF beta, Wnt5a and IL10 result in a functional phenotype of type "M2-like".

Similarly, *in vivo* studies have shown the existence of M1, M2 and M2-like macrophages. These subtypes represent only the extremes on a continuum of functional states that must be integrated in an environmental complex system.

Generally, M1 macrophages present IL12^{high}, IL23^{high} and IL10^{low} phenotype and produce molecular effectors such as reactive oxygen species (ROS) and intermediates of Nitric Oxide (NO) and inflammatory cytokines (IL1b, TNF-α, IL-6). M1 macrophages participate in Th1 responses, play a role in resistance against intracellular parasites and are key effectors in the elimination of tumor cells. In contrast, M2 macrophages have an IL12^{low}, IL-23^{low} and IL10^{high} phenotype with a variability in the production of inflammatory cytokines according to stimuli present in the environment. M2 cells display on their surface a strong expression of scavenger, mannose and galactose-type receptors. The metabolism of arginine is changed to an ornithine and polyamines metabolism. M2 macrophages are generally associated to a Th2 type response, to a parasite clearance, to a decrease of inflammation, to tissue repair promotion, angiogenesis, tumor growth and immune regulation.

M1 and M2 also have distinct expression profiles of chemokines. M1 macrophages express CXCL9 and CXCL10 chemokines which are known for attracting Th1, while M2 macrophages express CCL17, CCL22 and CCL24. Chemokines such as CCL2 and CXCL4 can also polarize macrophages to an M2-like phenotype.

Depending on their polarization state, macrophages have different characteristics in terms of iron, folate and glucose metabolisms. For example, M1 express large amounts of proteins involved in iron storage, such as Ferritin, while they express only weakly Ferroportin, involved in the iron exportation to the extracellular medium. In contrast, M2 macrophages express low levels of Ferritin but high levels of Ferroportin. This difference can result in functional outcomes, such as a bacteriostatic effect of M1 (protection against infection) and an effect promoting tissue repair by M2 macrophages, which also promote the tumor growth, as observed in some studies. The management of iron by macrophages according to their polarity is an important element underlining the importance of controlling the polarization of macrophages according to the condition of an individual.

Similarly, macrophages face an oxygen gradient in tissues under normal or pathological conditions. Macrophages or monocytes adapt to this gradient by modifying their glycolytic metabolism. The HIF1 and 2 are transcriptional factors leaders of these changes, including expression of chemokines or chemokine receptor CXCR4 or CXCL12 and VEGF (an angiogenic factor). Macrophages are involved in the tissue response to hypoxic conditions.

The presence of polyamines in the cell environment appears to be a type 2 macrophage polarization factor.

The present invention aims to modulate the polarization of macrophages in order to inhibit the anti-inflammatory M2-type macrophages and/ or to favor the pro-inflammatory M1-type macrophages.

### Tumor microenvironment

In the tumor environment, different defense cells exist. This is *a priori* a paradox because their presence should mean that they are attacking the tumor. In reality, many of these immune cells are maintained in an inactive stage and are rendered inoperative by the presence of regulatory cells. Instead of fighting the tumor, these regulatory cells facilitate tumor development by helping to overcome barriers, allowing it to spread and form secondary tumors, *i.e.,* metastases.

### Tumor immune escape and immune suppressive mechanisms

The cellular and molecular effectors of inflammation are important actors in the tumor microenvironment. Indeed, since the 90s, this interrelationship between inflammation and tumor is the subject of numerous studies (Mittal et al., 2014; Teng et al., 2015). To escape the immune system, tumor implements escape mechanisms in 3 stages: elimination - equilibrium - escape. The first phase is to eliminate immune mechanisms recognizing tumor cells. Then an equilibrium is set up between tumor cells and immunity: between killing and survive. This equilibrium can persist during years without the tumor progressing. During this period, tumor cells are subjected to genetic instability and eventually escape, inducing proper immune response or inhibiting the anti-tumor immune response by inducing said suppressor mechanisms, *i.e.,* blocking the anti-tumor response. The cells are then recognized as normal.

It is in this latter suppressor mechanism that inflammatory cells and molecules play an important role. The adaptive immune response is suppressed / blocked by activating a number of pathways leading to the inhibition of differentiation and activation of dendritic cells (via the presence in tumor microenvironment factors such as IL10 and VEGF). There is also an increase of the regulatory T cells (Treg) in peripheral blood and lymph nodes inhibiting innate and adaptive responses. The presence of tumor suppressor cells in the microenvironment, such as MDSC (myeloid derived suppressor cells) and TAM (Tumor Associated Macrophages or M2), affects tumor development of bad prognosis by the secretion of cytokines, growth factors, enzymes degrading the extracellular matrix and proteases (Cornelissen et al., 2012).

Immunotherapy is safe but in some cancer has a moderate efficacy partly due to the presence of immunosuppressive cells in peripheral blood, lymphoid organs and within the tumour environment that hamper immunotherapeutic treatments. Several strategies have been performed or are currently tested to improve the efficacy of immunotherapy by acting on suppressive cells such as MDSCs, Tregs and TAMs, which are increased in most cancer patients. It is becoming increasingly clear that these populations contribute to the impaired antitumour responses frequently observed in cancer patients.

Therefore, combating immunosuppression through modulation of these cell types is an important key to increase the efficacy of immunotherapy and should lead to a better prognosis for cancer patients. The present invention aims to modify the M1/M2 balance of macrophage population to favor the M1-type macrophages, in order to provide an immune environment propitious to immunotherapy.

### Macrophages and Cancer

Macrophages are found in large numbers in tumors. Originally it was thought that this cell population was involved in an anti-tumor response but many experimental and clinical studies have shown that macrophages are involved in the tumor initiation and progression as well as in the metastatic process. During the tumor process, macrophages secrete pro-inflammatory cytokines such as IFNy, TNF-α and IL6, attracting other immune cells creating chronic inflammation and causing the initiation and tumor progression. However, once the tumor installed, tumor macrophages (TAMs) adopt an immunosuppressive cellular profile and are less active, allowing tumor growth and transition to malignancy. TAMs are responsible for migration, extravasation and invasion of tumor cells (metastasis) and are involved in tumor angiogenesis (Qian and Pollard, 2010; DeNardo et al., 2010; Hanahan and Coussens, 2012).

Monocytes Ly6C⁺(CD14⁺)/CD11b^{high} arriving at the tumor undergo phenotypic changes such as a decrease of the Ly6C and CD11b markers and expression of MHC class II (MHCII), VCAM and CD11c. However, differentiation and distribution of TAMs depend on the tumor's anatomical localization and on its stage of development. The definition TAM's function was previously based on anti-tumor M1 macrophage type (iNOS inducible) and M2 pro-tumoral ARG-positive macrophage type. This simplistic dichotomy must be seen in a context of great plasticity of TAM in a cytokines and chemokines environment favoring their suppressive function and allowing the recruitment of Treg involved in tolerance to tumor cells (reviewed in Ugel et al., 2015; Wynn et al., 2013).

The present invention pertains to the inhibition of TAM in order to decrease or prevent the tumoral process, including the metastatic process.

### CD47-SIRPa pathway

Signal regulatory protein alpha, also termed CD172a or SHPS-1 and herein noted "SIRPa", was first identified as a membrane protein mainly present on macrophages and myeloid cells that was associated with the Src homology region 2 (SH2) domain - containing phosphatases - SHP-1 and SHP-2. SIRPa is the prototypic member of the SIRP paired receptor family of closely related SIRP proteins. Engagement of SIRPa by CD47 provides a downregulatory signal that inhibits host cell phagocytosis, and CD47 therefore functions as a "don't-eat-me" signal.

SIRPa is expressed on monocytes, most subpopulations of tissue macrophages, granulocytes, subsets of dendritic cells (DCs) in tissues, some bone marrow progenitor cells, and to varying levels on neurons, with a notably high expression in synapse-rich areas of the brain, such as the granular layer of the cerebellum and the hippocampus (Seiffert et al, 1994; Adams et al, 1998; Milling et al, 2010).

The SIRPa interaction with CD47 is largely described and provides a downregulatory signal that inhibits host cell phagocytosis (see review Barclay et al, Annu. Rev. Immunol., 2014). Both CD47 and SIRPa also engage in other interactions. Investigators have suggested that the lung surfactant proteins SP-A and SP-D control inflammatory responses in the lung through interactions with SIRPa (Janssen et al., 2008).

One of the best characterized physiological functions of CD47-SIRPa interactions is their role in the homeostasis of hematopoietic cells, in particular red blood cells and platelets. Because CD47 acts as a don't-eat-me signal and, as such, is an important determinant of host cell phagocytosis by macrophages, the potential contribution of CD47-SIRPa interactions in cancer cell clearance has been intensely investigated in recent years.

The SIRPa/CD47 pathway is nowadays also subject to different pharmaceutical developments, all directed towards enhancement of macrophages phagocytosis. In fact, like infected cells, cancer cells carry aberrant cargo such as unfamiliar proteins or normal proteins at abnormal levels, yet these cells frequently subvert innate immune control mechanisms by concurrently over-expressing immunoregulatory molecules. It is becoming increasingly clear that one such mechanism involves CD47 (Barclay and Van den Berg, 2014), a protein of "self" expressed by normal cells. CD47 has interactions with several different ligands such as SIRPa. This specific interaction is known to lead to a "don't eat me" signal to phagocytic macrophages, which then leave target cells unaffected (Oldenborg et al., 2000) Over-expression of CD47 by cancer cells renders them resistant to macrophages, even when the cancer cells are coated with therapeutic antibodies (Zhao et al., 2011), and correlates with poor clinical outcomes in numerous solid and hematological cancers (Majeti et al., 2009; Willingham et al., 2012). In experimental models, in particular human tumor-xenograft models in immunodeficient mice, blockade of the CD47/SIRPa pathway was very effective to promote tumor elimination by macrophages and to decrease cancer cell dissemination and metastasis formation (Chao et al., 2011; Edris et al., 2012; Uluçkan et al., 2009; Wang et al., 2013). In these studies, TAM function or phenotype has not been studied. Blockade of the CD47/SIRPa pathway, by enhancing antibody-dependent phagocytosis by macrophages, has been described to synergize with depleting therapeutic anticancer antibodies (Weiskopf et al., 2013) such as Trastuzumab (anti-Her2), Cetuximab (anti-EGFR), Rituximab (anti-CD20) and Alemtuzumab (anti-CD52).

From the above, it appears that SIRPa has been described to be implicated into the phagocytic function of myeloid cells, the antigen presentation and cytokine secretion of dendritic cells and trafficking of mature granulocytes. However, the function of SIRPa on macrophage polarization and their potent suppressive function during tumorigenesis have never been described.

WO2010/130053 disclosed a method for treating hematological cancer comprising modulating the interaction between human SIRPa and CD47. This document showed that the blockade of SIRPa-CD47 induces the activation of the innate immune system via the phagocytosis pathway. In the transplantation model of human leukemia described in this patent application, myeloid cells were used and the transplant was rejected when animals were treated with an antagonist of CD47. This result suggests an increase of phagocytosis upon treatment with anti-CD47, but not a modification in the polarization state of the macrophages nor a modification into a pro-inflammatory function of the macrophages.

A method for inhibiting cell functioning for use in anti-inflammatory and anti-tumor therapies was described in WO0066159. This method comprises administering a drug comprising a substance that specifically recognizes the extracellular domain of SIRPa and that inhibits the functioning of pathologic myeloid cells. The inventors of this patent application claim that an anti-SIRPa antibody specific to the extracellular domain has the property to block the inflammation and inhibit macrophage phagocytosis (referred to as the functioning of pathologic myeloid cells in the text). This effect is in total contradiction with the results disclosed below and do not suggest any effect of an anti-SIRPa antibody on the macrophage polarization nor on pro-inflammatory function.

A CD47-Fc and a CD47-extended fusobody molecules that bind to SIRPa were studied and claimed in the patent application number WO2012/172521. These molecules have been claimed to be able to inhibit immune complex-stimulated cell cytokines release (e.g., release of IL-6, IL10, IL12p70, IL23, IL8 and TNF-α) from peripheral blood monocytes, DCs and/or monocytes-derived DCs stimulated with Pansorbin or soluble CD40L and IFNy. The activity of these molecules is completely different from the activity of anti-SIRPa antibodies disclosed in the present application.

WO2013/056352 describes the use of an antibody anti-human SIRPa (called 29AM4-5, and corresponding to SIRP-29 as named by the inventors of the present invention) in a model of SIRPa-positive AML, i.e. xenotransplantation into immunodeficient mice of primary human AML cells expressing human SIRPa to which bind said antibody anti-human SIRPa. Thus, the approach consists in the use of an anti-human SIRPa antibody to act on tumor cells expressing human SIRPa. The treatment is thus directly directed toward the tumor.

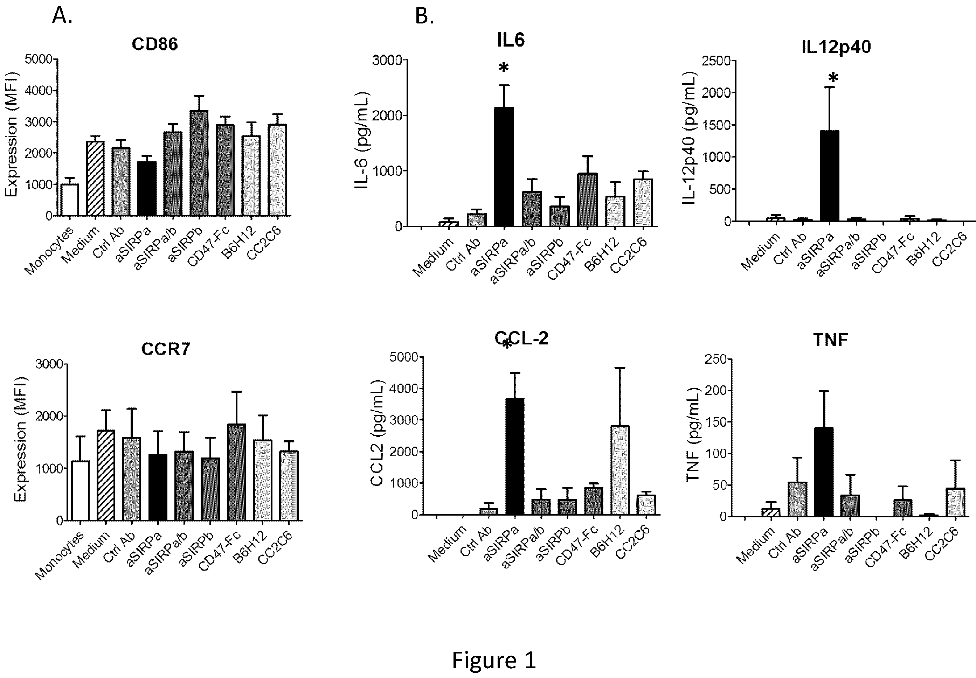

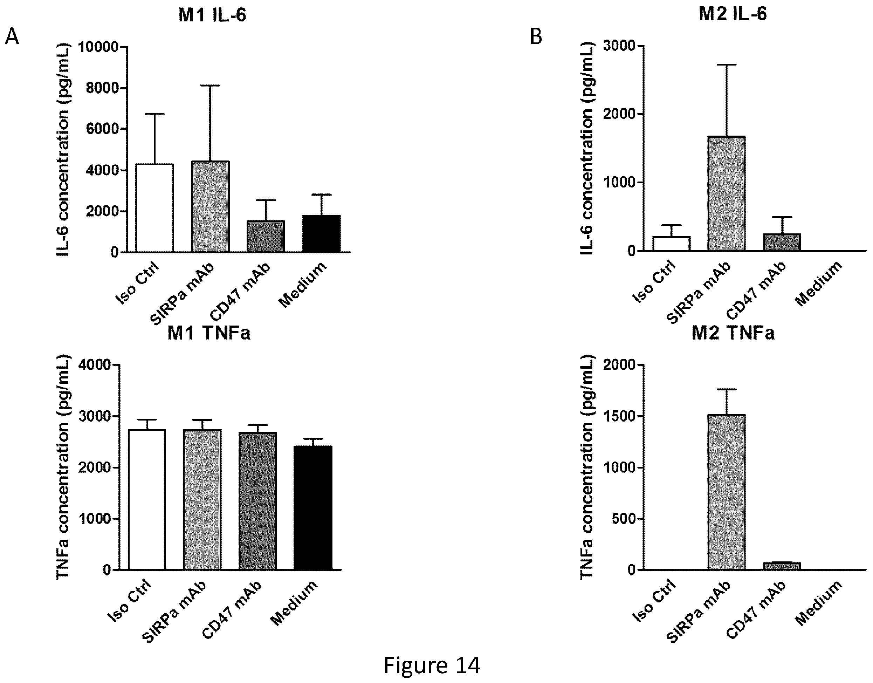

Alblas J. et al (2005, Molecular and Cellular Biology) observed that an anti-SIRPa antibody (ED9) is not able to induce to the production of inflammatory cytokines, especially TNF-α and IL-6 (Figure 1A). However, the inventors of the present invention demonstrated, on the contrary, that this particular antibody does allow to repolarize M2 anti-inflammatory macrophages into M1 pro-inflammatory macrophages (Figure 14B of the experiment part). The difference of result could be explained by the fact the authors of Alblas *et al.* used a macrophage cell line (rat NR8383 cell line) (which may no longer express SIRPa), whereas the inventors used a fresh preparation of rat macrophage derived from bone marrow in the course of the experiment.

WO 2015/138600 describes anti-human SIRPa antibodies that bind to human SIRPa and block the interaction with CD47 expressed on a target cell with SIRPa expressed on a phagocytic cell. This document does not suggest nor show any evidence of the effect of an anti-SIRPa on macrophage polarization nor on the increase of the pro-inflammatory function of macrophages. Interestingly, the inventors named in WO 2015/138600 published an abstract at the 56th ASH annual meeting (Weiskopf et al, ASH 2014). In this abstract, which is posterior to the filing of WO 2015/138600, they mentioned that anti-human SIRPa antibodies blocking the interaction between CD47 and SIRPa were not sufficient to induce human macrophage phagocytosis. Altogether, these publications suggest that the mechanism of action of an anti-human SIRPa *in vivo* is different from that of an anti-human CD47, for which the *in vivo* phagocytic efficacy was largely described in the literature.

From the above, it appears that various blocking SIRPa-CD47 interaction strategies targeting CD47 or SIRPa with an antibody or a fusion protein show distinct results and different efficiencies, indicating distinct roles for each of the targets. Indeed, an antibody directed against the CD47 antigen blocks the interaction of CD47 with all its ligands. The use of a SIRPa-Fc protein that binds to the cells through its Fc and blocks the endogenous CD47 pathway and prevents its activation is not able to block the activity of endogenous SIRPa, contrary to an anti-SIRPa antibody. The direct role of the SIRPa pathway in modulating the immune system has been so far undervalued compared to the CD47 pathway. None of the prior art studies suggests nor describes any function of the SIRPa pathway in the polarization of macrophages (either at a phenotype level or at a functional level) that plays a crucial role in the tumor escape mechanisms.

In this context, the inventors provide a new insight in the use of anti-SIRPa compounds since the modulation of the immune environment is a major achievement in the treatment of many diseases, especially in cancer.

### SUMMARY OF THE INVENTION

As described in the experimental part below, the inventors have now identified SIRPa as a new checkpoint inhibitor and demonstrated its role in macrophage polarization. They indeed showed that an anti-SIRPa compound as defined herein, induces a pro-inflammatory function of macrophages associated to type 1 macrophages (M1 pro-inflammatory = M (IFNg)) and inhibits the suppressive activity of M2-type macrophages in the tumor, since the pro-inflammatory profile of macrophages is obtained at the expense of type 2 macrophages (M2 type high phagocytic activity = M (IL4)). This effect is obtained by targeting SIRPa but not CD47, which is involved in phagocytosis function. One advantage of the invention is that the use of an anti-SIPRa compound will induce less side effects than the use of an anti-CD47 compound. Indeed, CD47 is expressed by a large range of cells, and not only by tumor cells and interacts with several ligands. The expression of SIRPa being more limited, the effect of the therapy will be more targeted on the tumor microenvironment. Thus, the use of an anti-SIRPa compound will be less toxic and less deleterious than the use of an anti-CD47 compound. Further, the therapy being directed toward macrophages and not tumor cells, no selection pressure will be exercised on tumor cells allowing to prevent tumor escape and the development of tumor resistance to the treatment.

The present invention hence pertains to the use of an anti-SIRPa compound able to inhibit the polarization of anti-inflammatory M2-type macrophages and/or favors pro-inflammatory M1-type macrophage, such as an anti-SIRPa antibody, for modifying macrophage polarization. The method of the invention thus consists in the use of an anti-SIRPa compound wherein said compound inhibits polarization of M2-type macrophages and/or favors pro-inflammatory M1-type of macrophages.

In a particular embodiment, said anti-SIRPa compound can be selected from the group consisting of an anti-SIRPa antibody, in particular an anti-SIRPa antagonist antibody, a nucleic acid encoding such compound, and a compound able to inhibit the expression of the SIRPa protein, in particular a siRNA.

Anti-SIRPa compounds, in particular anti-SIRPa specific antibodies, can thus be used in the treatments of various conditions likely to be improved or prevented by pro-inflammatory macrophages, such as cancers, infectious diseases, traumas, auto-immune diseases, vaccination, neurologic diseases, brain and nerve injuries, polycythemias, hemochromatosis and chronic inflammatory diseases. Cancer is a preferred therapeutical indication.

In a particular embodiment, the present invention concerns an anti-SIRPa compound able to inhibit the polarization of anti-inflammatory M2-type macrophages and/or favors pro-inflammatory M1-type macrophage for use in the treatment of cancer, with the exception of SIRPa-positive acute myeloid leukemia and/or SIRPa-positive non acute myeloid leukemia and/or SIRPa-positive non-Hodgkin leukemia or SIRPa-positive hematologic cancers.

An important aspect of the invention is that the therapeutical approach aims to target SIRPa on the macrophage in order to modulate their polarization and to recreate an immune context detrimental to the tumor development and survival. Basically, the success on the anti-tumor treatment is based on an indirect pathway, and does not require that the tumor cells are sensitive to the anti-SIRPa compound. Accordingly, in a particular embodiment, the present invention concerns the use of an anti-SIRPa compound as defined herein, i.e. able to inhibit the polarization of anti-inflammatory M2-type macrophages and/or favors pro-inflammatory M1-type macrophage, in the treatment of cancer, wherein said compound is administered to a patient presenting a SIRPa-negative tumor.

Anti-SIRPa compounds as defined herein can be used in monotherapy as described in the experimental part.

Combinations of anti-SIRPa compounds as defined herein with other therapeutic agents, especially with agents blocking another immune checkpoint, are also part of the invention, since a synergistic effect was demonstrated by the inventors.

Another aspect of the present invention is a method for *ex vivo* obtaining pro-inflammatory M1-type macrophages by incubating macrophages with an anti-SIRPa compound as defined herein.

The present invention also pertains to a method for selecting patients likely to benefit from a treatment by an anti-SIRPa compound as defined herein, by measuring the presence of M2-type macrophages in a sample from the patient.

A method of following-up a treatment by an anti-SIRPa compound as defined herein, to assess its efficacy by measuring the presence of pro-inflammatory M1-type macrophages and/or measuring the presence of anti-inflammatory M2-type macrophages in a sample from an individual treated by said compound, is also part of the present invention.

### LEGENDS TO THE FIGURES

**Figure 1** **: Monocyte type 1 polarization with GM-CSF + M-CSF protocol: Effect of SIRPa blockade:** Human Monocytes were cultivated with growth factors M-CSF and GM-CSF to induce Macrophages and treated or not with Ctrl Ab or anti-Sirp antibodies (anti-a or ab or b isotype), or a CD47-Fc protein, or some anti-CD47 antibodies (B6H12 or CC2C6). Then type 1 macrophage phenotype and function were analysed by FACS and ELISA. A. represents cell surface markers analysis: CD86 and CCR7. B. represents secreted cytokine/chemokine: IL6, IL12p40, CCL-2 and TNF-α. IL6, p12p40 and CCL-2 are significantly increased when cells are treated with an anti-SIRPa antibody.

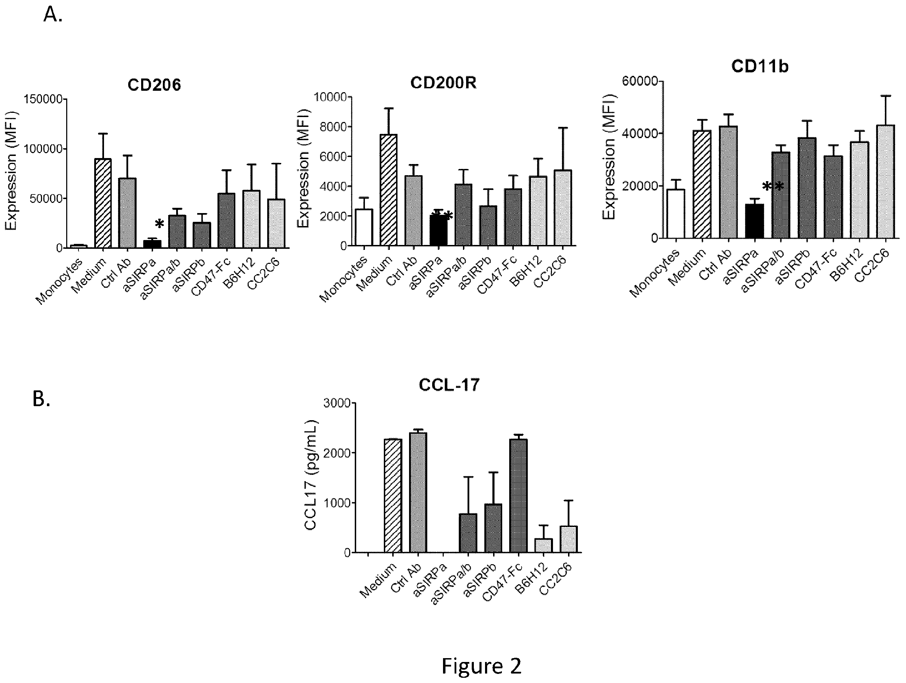

**Figure 2** **: Monocyte Type 2 polarization with GM-CSF + M-CSF protocol : Effect of SIRPa blockade:** Human Monocytes were cultivated with growth factors M-CSF and GM-CSF to induce macrophages and treated or not with Ctrl Ab or anti-Sirp antibodies (anti-a or ab or b isotype), or a CD47-Fc protein, or some anti-CD47 antibodies (B6H12 or CC2C6). Then Type 2 macrophage phenotype and functional analysis were performed by FACS and ELISA. A. Shows marker expression at cell surface : CD206, CD200R, CD11b and B. Shows secreted Chemokine : CCL-17

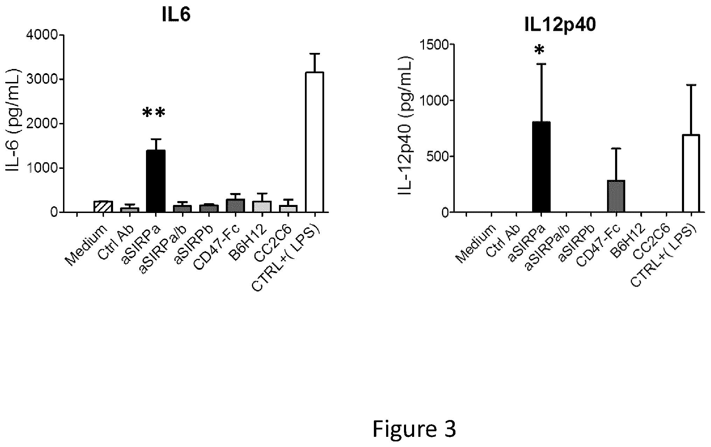

**Figure 3**: **Monocyte type 1 polarization with M-CSF and IFNgamma protocol: Effect of SIRPa blockade** : Human monocytes were cultivated 5-6 days with M-CSF and then 2 days with IFNgamma with or without Ctrl Ab or anti-Sirp antibodies (anti-a or ab or b Sirp isotype), or a CD47-Fc protein, or some anti-CD47 antibodies (B6H12 or CC2C6). M1 secreted cytokines were analysed : IL6 and IL12p40. Statistical analysis were performed

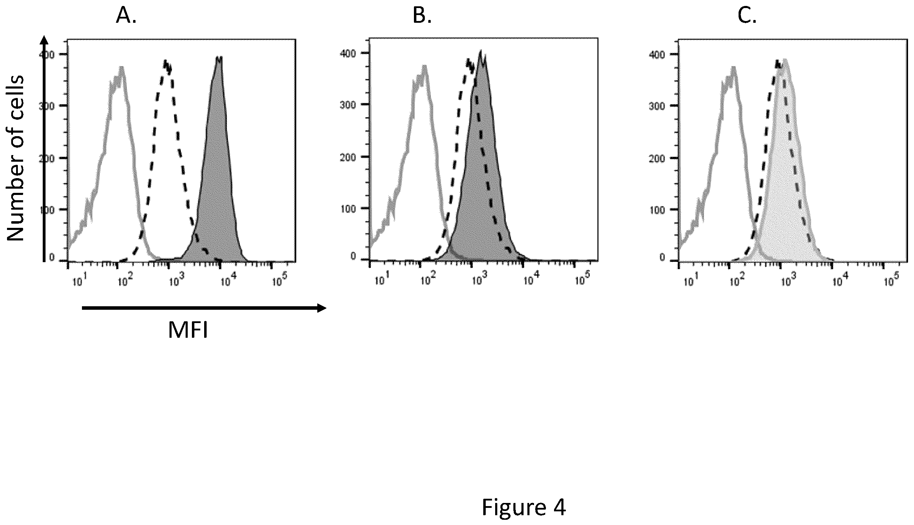

**Figure 4****: Monocyte type 1 polarization with M-CSG + IFNgamma and LPS protocol: Effect of SIRPa blockade on iNOS expression:** Human monocytes were cultivated 5-6 days with M-CSF and then 2 days with IFNgamma+LPS with or without antibodies directed against : SIRPa: SE7C2 (A.) or SIRPa/b: SE5A5 (B.) or CD47: B6H12 (C.), iNOS expression was then analysed by FACS. Ctrl Ab is represented by dotted line and monocytes before polarization by empty grey line on the three panels

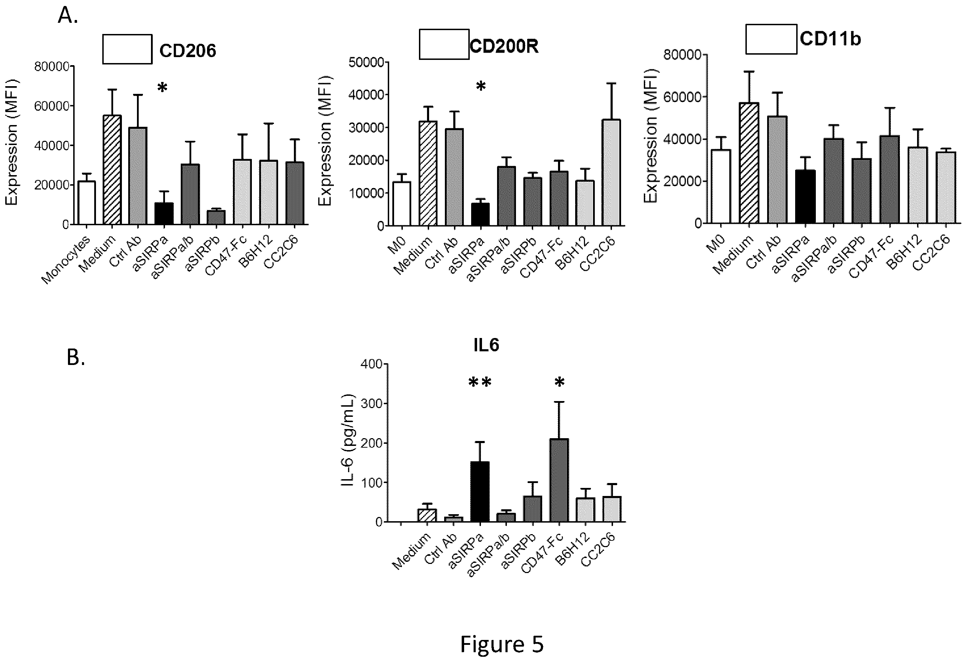

**Figure 5****: Monocyte type 2 polarization with M-CSF + IL4 protocol: Effect of SIRPa blockade with antibody** : Human monocytes were cultivated 5-6 days with M-CSF and then with IL4 with or without Ctrl Ab or anti-Sirp antibodies (anti-sirp a or ab or b), or a CD47-Fc fusion protein, or some anti-CD47 antibodies (B6H12 or CC2C6). A. Represents expression of cell surface markers: CD206, CD200R and CD11b and B. Represents the release of IL6 cytokine in the supernatant

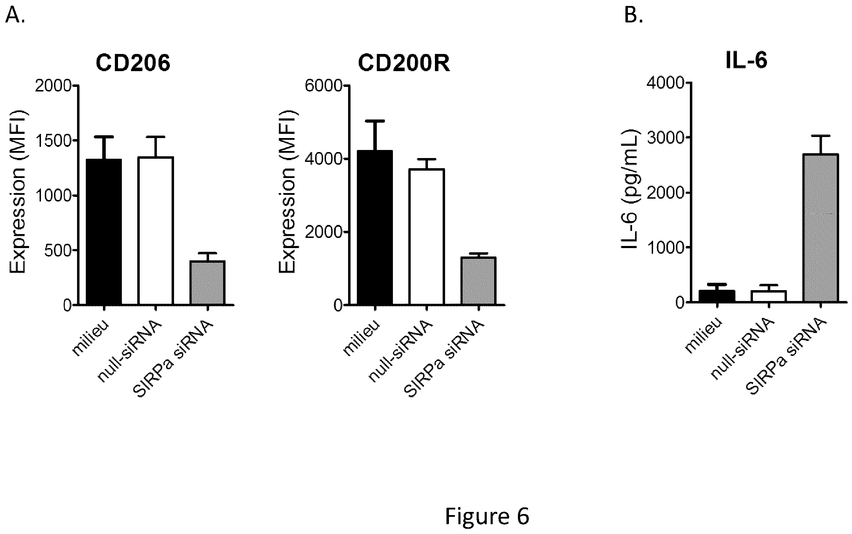

**Figure 6** **: Monocyte type 2 polarization with M-CSF + IL4 protocol: Effect of SIRPa blockade at a mRNA level with a siRNA Knock down assay:** Human monocytes were cultivated 5-6 days with M-CSF and then 2days with IL4. Cells were transfected with null-siRNA or SIRPa siRNA : A. represents the expression of the cell surface markers : CD206 and CD200R; B. represents the IL6 cytokine release in the supernatant. The anti-SIRPa SiRNA are provided by ThermoFisher scientific.

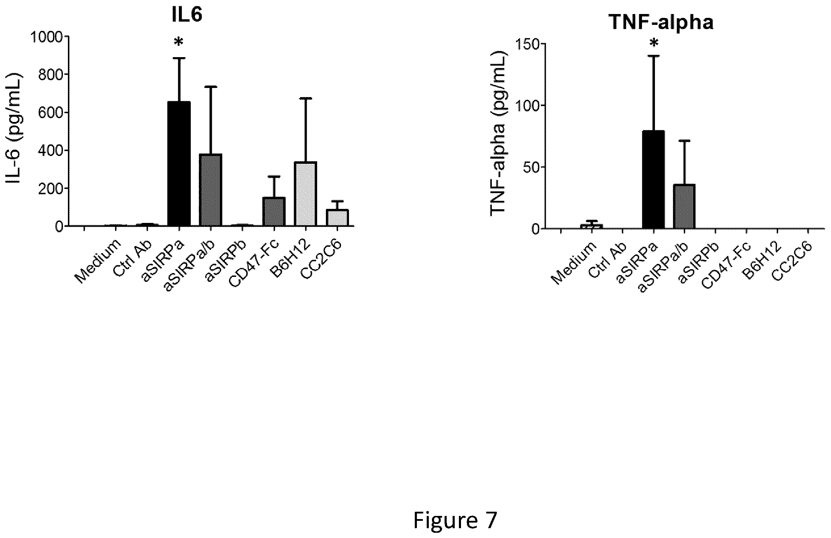

**Figure 7** **: Effect of SIRPa blockade on M2 repolarization after M-CSF+IL4 treatment:** Human monocyte were cultivated 5-6 days with M-CSF and then 2 days with IL4. M2 macrophages were then treated with or without Ctrl Ab or anti-Sirp antibodies (anti-sirp a or ab or b), or a CD47-Fc fusion protein, or some anti-CD47 antibodies (B6H12 or CC2C6). The Cytokines IL6 and TNF-α releases were then measured in the supernatant.

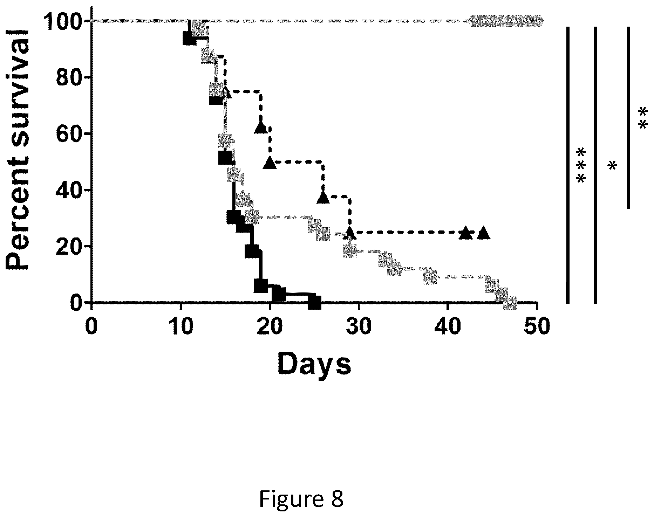

**Figure 8** **: Effect of a combined anti-SIRPa + anti-CD137 treatment on an in vivo model of Hepatocarcinoma:** One week after tumor inoculation, animals were treated 3 times/week for 4 weeks with 3G8 isotype control antibody (Iso ctrl: black square: n=33), or an anti-SIRPa antibody (clone p84 :grey square; n= 33) or an antiCD137 antibody (4-1BB mAb: black triangle; n=8) or a combined treatment (Anti-SIRPa+anti-CD137: grey diamond; n=8). The overall survival rate was then analyzed.

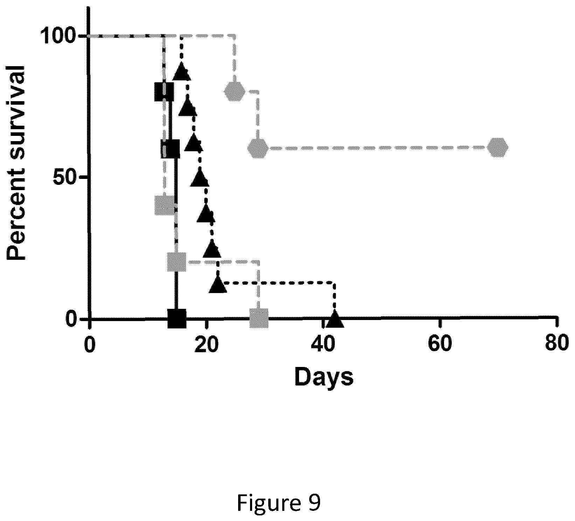

**Figure 9****: Effect of a combined anti-SIRPa + anti-PD-L1 treatment on an in vivo model of Hepatocarcinoma:** One week after tumor inoculation, animals were treated 3 times/week for 4 weeks, animals were treated with 3G8 isotype control antibody (Iso ctrl: black square: n=5), or an anti-SIRPa antibody (clone p84: grey square; n= 5) or an anti-PD-L1 antibody (10F-9G2 mAb: black triangle; n=8) or a combined treatment (anti-SIRPa+anti-PD-L1: grey diamond; n=5). The overall survival rate was then analyzed.

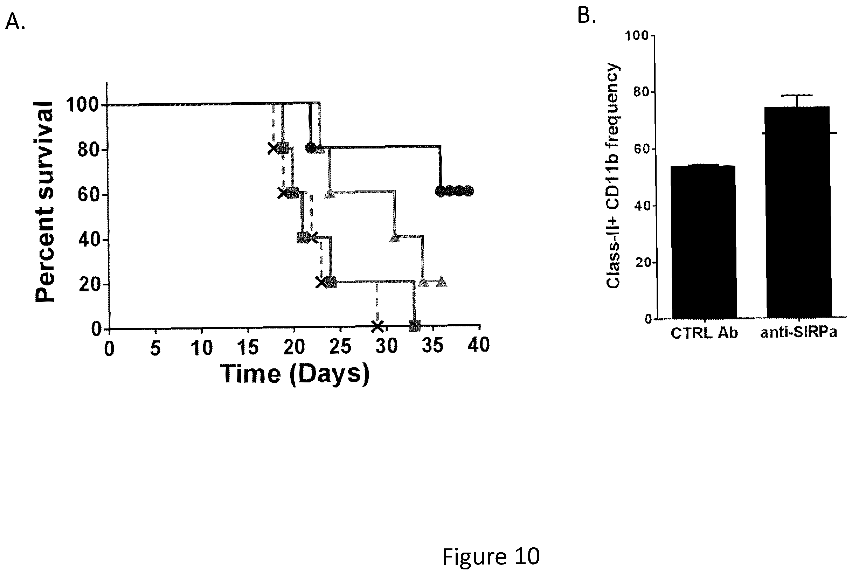

**Figure 10** **: Effect of a combined anti-SIRPa + anti-PD-L1 treatment on an in vivo model of melanoma:** In the same time as tumor inoculation, animals were treated with isotype control antibody (Iso ctrl: star: n=5), or an anti-SIRPa antibody (clone p84 : square; n= 5) or an anti-PD-L1 antibody (10F-9G2 mAb: triangle; n=8) or a combined treatment (anti-SIRPa+anti-PD-L1: circle; n=5).A. The overall survival rate was then analyzed. B. Some animals were sacrificed 2 weeks after first inoculation to analyze macrophage infiltrate using CMH class II/CD11b markers.

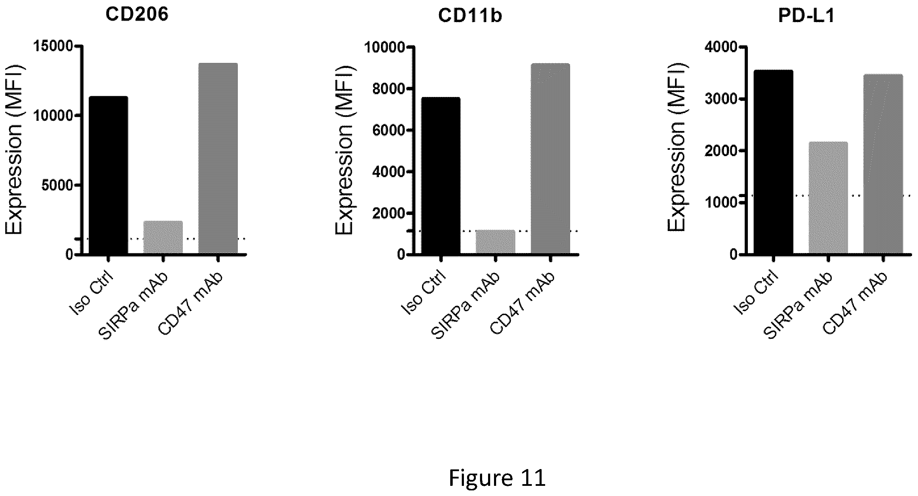

**Figure 11** **: Effect of anti-SIRPA** antibody on mouse macrophage polarization: Mouse M2 macrophage polarization phenotype is inhibited by anti-SIRPa mAb but not by anti-CD47 mAb as shown by the quantification of the expression of the M2 markers CD206, CD11b and PD-L1. In this experiment, anti-SIRPa mAb corresponds to p84 clone and anti-CD47 mAb corresponds to MIAP310 clone.

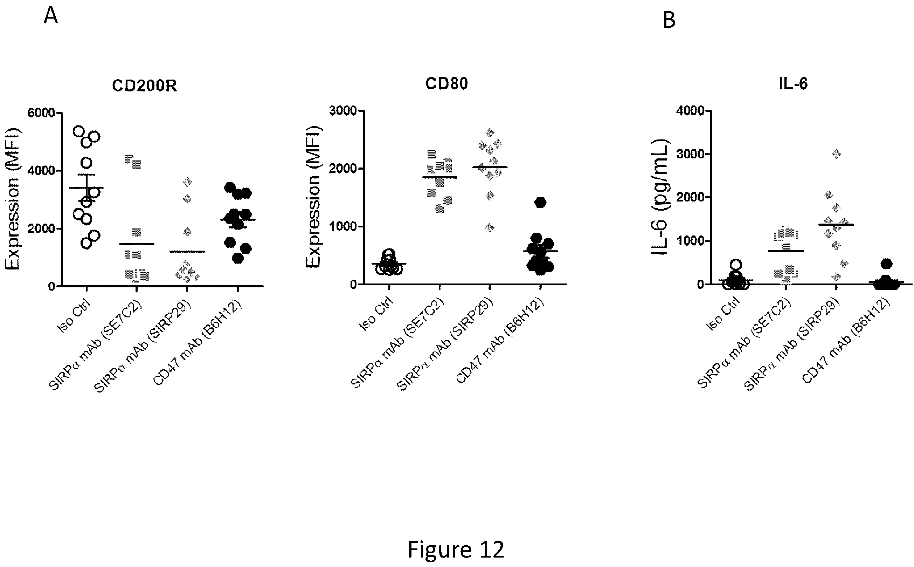

**Figure 12** **: Effect of anti-SIPRa antibody on human M2 macrophage polarization:** Human M2 macrophage polarization phenotype is inhibited by anti-SIRPa mAbs but not with anti-CD47 mAb. A. By detection of M2 surface markers (CD200R, CD80). B. At functional level by evaluation of cytokine secretion (IL-6). In this experiment, anti-SIRPa mAbs correspond to SE7C2 or SIRP29 clones and anti-CD47 mAb corresponds to B6H12 clone.

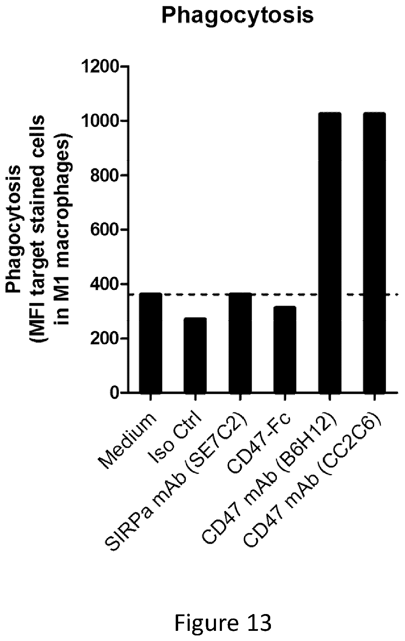

**Figure 13** : **Effect of anti-SIPRa antibody on human M1 phagocytosis:** Human macrophage phagocytosis of CD47+ tumor cells (Raji) is increased by CD47 targeting agents, but not with SIRPa targeting agents. Analysis of phagocytosis was evaluated by flow cytometry by gating on M1 fluorescent macrophages and analysis of target (Raji) cells fluorescence into M1 macrophages. In this experiment, the CD47 targeting agents correspond to anti-CD47 mAbs B6H12 or CC2C6 clones and the SIRPa targeting agents correspond to anti-SIRPa clone SE7C2 or CD47-Fc protein.
**Figure 14** **: Effect of anti-SIRPA antibody on rat M1/M2 polarization:** A. Rat M2 macrophages become pro-inflammatory and secrete inflammatory cytokines (IL-6 and TNF-α) in presence of anti-SIRPa mAb, but not anti-CD47 mAb. B. M1 rat macrophages polarization is not modified by anti-SIRPa or anti-CD47 mAbs. Anti-SIRPa mAb used in this experiment corresponds to ED9 clone and anti-CD47 mAb corresponds to OX 101 clone.

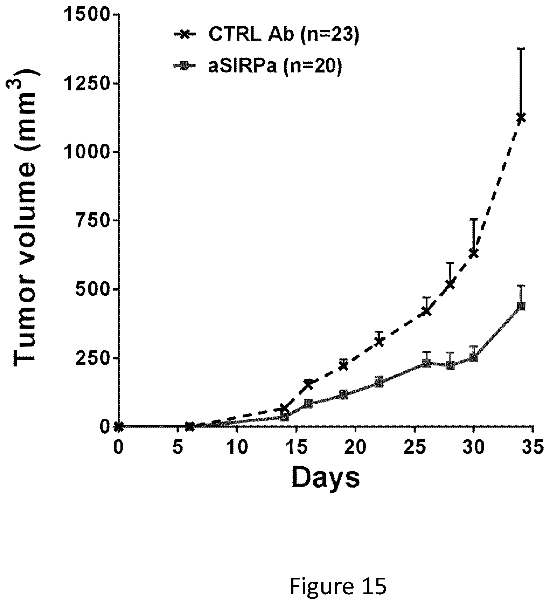

**Figure 15** **: Effect of anti-SIRPa antibody on a mouse model of mammary tumor.** Monotherapy with anti-SIRPa mAb inhibit tumor growth in the 4T1 mammary cancer model in mice. Anti-SIPRa mAb used in this experiment corresponds to clone p84.

### DETAILED DESCRIPTION OF THE PREFERRED EMBODIMENTS

Throughout the present text, the following definitions are used:

### Macrophage polarization

The term "polarization" is used herein to designate the phenotypic features and the functional features of the macrophages. The phenotype can be defined through the surface markers expressed by the macrophages. The functionality, can be defined for example based on the nature and the quantity of chemokines and/or cytokines expressed, in particular secreted, by the macrophages. Indeed, the macrophages present different phenotypic and functional features depending of their state, either pro-inflammatory M1-type macrophage or anti-inflammatory M2-type macrophage. M2-type macrophages can be characterized by the expression of surface markers such as CD206, CD11b, PD-L1 and CD200R and then secretion of cytokines such as CCL17. M1-type macrophages can be defined by the expression of surface markers such as CD86 and CCR7 and the secretion of cytokines such as IL-6, TNF-α and IL12p40. In the context of the invention, anti-SIPRa compounds allow to modulate the polarization of macrophages population by inhibiting the M2-type macrophages and/or favoring the M1-type macrophages.

It encompasses the meaning of the term "activation" usually used to mean the perturbation of macrophages with exogenous agents. Macrophages change their polarization states in response to growth-factors (CSF-1 and GM-CSF) and external stimuli such as microbes, microbial product and nucleotides derivatives, antibody-Fc receptor stimulation, glucocorticoids, phagocytosis.

### Anti- SIRPa compound

An "anti-SIRPa compound", as used herein refers to a compound that specifically binds to the extracellular domain of SIRPa, or a nucleic acid encoding such compound, as well as to a compound able to inhibit the expression of the SIRPa protein. Such compound is able to inhibit the polarization of anti-inflammatory M2-type macrophages and/or favors pro-inflammatory M1-type macrophage.

An anti-SIRPa compound can be a compound specifically binding to the extracellular domain of the signal regulatory protein alpha (SIRPa). An anti-SIRPa compound can also be a SIRPa-blocking compound. Preferably, an anti-SIRPa compound is an antagonist peptide or an anti-SIRPa antibody, in particular an anti-SIRPa antagonist antibody.

As used herein, an antibody refers to polyclonal antibody, monoclonal antibody or recombinant antibody. The monoclonal antibodies of the present invention include recombinant antibodies for example, chimeric, CDR graft and humanized antibodies, but also antigen-binding moiety. As used herein, the term "antigen-binding moiety" of an antibody (or simply "antibody moiety") refers to one or more fragments of an antibody of the invention, said fragment(s) still having the binding affinities as defined above. In accordance with the term "antigen-binding moiety" of an antibody, examples of binding fragments include Fab fragment, F(ab')2 fragment, Fv fragment, and single chain Fv (ScFv). Other forms of single chain antibodies such as "diabodies" are likewise included here.

As used herein, an antagonist peptide refers to a peptide able to inhibit the interaction of SIRPa with one of its ligands, especially with CD47, or to a peptide able to prevent or decrease the SIRPa signaling pathway.

An anti-SIRPa compound can also be a compound able to inhibit the expression of the SIRPa protein such as an antisens oligonucleotide, or an interfering RNA such as a shRNA or a siRNA. Examples of siRNA able to modulate the polarization of the macrophages according to the invention are provided in the experimental part. Further, a skilled person in the art knows how to identify such compound.

In a particular embodiment, an anti-SIRPa compound able to inhibit the polarization of anti-inflammatory M2-type macrophages and/or favors pro-inflammatory M1-type macrophage can be identified by applying one of the protocols described in the experimental part below.

For example, the identification of an anti-SIRPa X compound can be performed as follows:
M0 macrophages are obtained by culturing monocytes 5 to 6 days with M-CSF (100µg/mL), then cells are cultured *in vitro* during 2 days with recombinant hIL4 (20ng/mL) in the presence or in the absence of said compound X. As a positive control of anti-SIRPa activity, anti-SIRPa mAb with a known activity can be used. Any compound not targeting the extracellular domain of SIRPa can be used as a negative control (*e.g.,* an anti-SIRPb antibody or a CD47-Fc). The secretion of different cytokines, for example IL-6, TNF-α and IL12 and chemokine CCL17 can be measured by ELISA.

The compound X can be classified as an "anti-SIRPa compound" if (i) it inhibits hallmarks of M2 macrophage features, in particular the overexpression of surface markers such as CD206, CD11b, PD-L1 and/or CD200R, and the secretion of specific cytokines such as CCL17 and/or (ii) it increases hallmarks of M1 macrophage features, in particular the expression of surface markers such as CD86 and CCR7 and the secretion of cytokines such as IL-6, TNF-α, IL12-p40 and/or IL12, as efficiently or more efficiently than the positive control mAb.

In a particular embodiment, the effect of an anti-SIRPa compound can be evaluated in comparison with the effect of the particular antibody SE7C2 (Santa Cruz sc-23863), used at a concentration of 10 µg/mL. The compound X is classified as an "anti-SIRPa compound" if it the results of the testing show that it is as efficient as SE7C2, or more efficient than SE7C2.

Based on this protocol, the comparison can be done with any anti-SIRPa compound of reference, as a positive control.

### Cancer, treatment, etc.

As used herein, "cancer" means all types of cancers. In particular, the cancers can be solid or non-solid cancers. Non limitative examples of cancers are carcinomas or adenocarcinomas such as breast, prostate, ovary, liver, lung, bladder, pancreas or colon cancer, sarcomas, lymphomas, melanomas, leukemias, germ cell cancers and blastomas.

As used herein, the terms "treat", "treatment" and "treating" refer to any reduction or amelioration of the progression, severity, and/or duration of cancer, particularly a solid tumor; for example in a liver cancer, reduction of one or more symptoms thereof that results from the administration of one or more therapies.

### Therapeutic agent

As used herein, a "therapeutic agent" designates any active force or substance capable of producing an effect. Therapeutic agents thus include radiations, surgery, probiotics as well as any kind of drug.

### Immune checkpoint blockers and stimulators

In the present text, a "drug blocking an immune checkpoint", or "immune checkpoint blocker" or "immune checkpoint blockade drug" or "immune checkpoint inhibitor" designates any drug, molecule or composition which blocks an immune checkpoint. In particular, it encompasses an anti-CTLA-4 antibody, an anti-PD-1 antibody, an anti-PD-L1 antibody and an anti-SIRPa antibody. Further, two or more immune checkpoint inhibitors can be combined to target both adaptive or innate immune cells. This approach is particularly interesting in the treatment of cancer. With this aim, an anti-SIRPa compound can be combined with an anti-PD-L1 compound. Such combinations can be used simultaneously separately or sequentially, particularly, in the treatment of cancer.

On the contrary "immune checkpoint stimulator" designates any drug, molecule or composition which activates an immune checkpoint. Such molecules or drugs that stimulate costimulatory molecules, resulting in target cell activation are for example anti-CD 137 antibodies.

Other definitions will be specified below, when necessary.

A first aspect of the present invention is the use of an anti-SIRPa compound able to modify macrophage polarization, in particular able to inhibit the polarization of anti-inflammatory M2-type macrophages and/or favors pro-inflammatory M1-type macrophage, in an individual (e.g., a human) in need thereof. As described above, macrophages mature in tissues and are activated in a dynamic response to combinations of stimuli to acquire specialized functional phenotypes that, in certain cases, are detrimental to the individual. As for the lymphocyte system, a dichotomy has been proposed for macrophage activation: classic (M1) vs. alternative (M2). Although several intermediate functional states have been observed, M1 and M2 subtypes remain relevant to describe the extremes on a continuum of macrophage states, M1 being the most pro-inflammatory state and M2 being more associated with a decrease of inflammation.

By "modifying macrophage polarization", it is herein meant that the compound modifies the balance between the different subtypes of macrophages in the treated individual, at least at the phenotypic and/or functional level. Hence, according to the present invention, the treatment of an individual with an anti-SIRPa compound leads to a modification in the profile of surface markers expressed by the individual's macrophage (including a decrease of CD206, CD11b, PD-L1 and/or CD200R expression and an increase of CD86 and CCR7 expression) and the profile of chemokines and cytokines expressed and/or secreted by the individual's macrophages (including a decrease of CCL-17 expression and an increase of IL6, TNF-α and IL12p40 expression).

According to a particular embodiment of the present invention, the modification of macrophage polarization by the anti-SIRPa compound includes an inhibition of M2-type macrophage polarization and/or an increase of pro-inflammatory M1-type macrophage polarization. In particular, it can include an inhibition of M2 phenotypic polarization of macrophages, leading to a decrease of the proportion of macrophages overexpressing cellular markers such as CD206, CD11b, PD-L1 and/or CD200R and/or producing cytokines such as CCL17. Concomitantly, the anti-SIRPa compound can induce the emergence of more macrophages exhibiting a M1 phenotypic polarization and an increase of the proportion of macrophages overexpressing cellular markers such as CD86 and CCR7 and/or producing cytokines such as IL-6, IL-12p40 and/or TNF-α. The anti-SIRPa compound can thus modulate the macrophage polarization at both phenotypic level (expression of cellular surface markers) and functional level (production of chemokines and cytokines).

According to a preferred embodiment of the present invention, the compound used for modifying macrophage polarization is an anti-SIRPa compound. An exemplary test to determine if a given compound is an anti-SIRPa compound in the sense of the present application is described above.

Among the compounds which can be used according to the present invention, one can cite small chemical molecules, polypeptides, antagonist peptides, antibodies and fragments thereof, especially anti-SIRP antibodies such as those used in the experiments described below, any other antibody selected amongst the many anti-SIRPa commercially available antibodies or any other (new) anti-SIRPa antagonist antibody, fragments of antibodies, aptamers targeting SIRPa, *etc.*

The present invention also pertains to the use of a nucleic acid (mRNA or DNA) encoding an anti-SIRPa compound, for modifying macrophage polarization to favor M1 pro-inflammatory macrophages in an individual in need thereof. Indeed, administering a nucleic acid which leads to the expression of an anti-SIRPa compound, as above-described by the patient's cells is an alternative possibility for obtaining the desired effect on macrophage polarization. The skilled artisan is free to choose any expression cassette with any regulatory elements, as well as any vectors (polymer, lipidic vectors such as cationic and/or liposome or viral vectors such as adenovirus, lentivirus, adeno associated virus (aav)) to obtain the expression of the anti-SIRPa compound at an appropriate level in an appropriate number of the patient's cells. In the following description of the detailed embodiments of the invention, the fact that a nucleic acid enabling the expression of an anti-SIRPa can be used instead of the anti-SIRPa compound itself will not be repeated. The term "anti-SIRPa compound" is herein considered to encompass nucleic acids enabling the *in vivo* expression of such a compound.

Particularly, the anti-SIRPa compound is selected from the group consisting of an antagonist peptide, an anti-SIRPa antibody, in particular an anti-SIRPa antagonist antibody, a nucleic acid encoding such compound, and a compound able to inhibit the expression of the SIRPa protein, in particular a siRNA. Preferably, an anti-SIRPa compound is an antagonist peptide or an anti-SIRPa antibody, in particular an anti-SIRPa antagonist antibody.

Modifying the polarization of macrophages to favor M1 pro-inflammatory cells can be useful in a number of pathologies or situations. As described above, this modification is particularly useful in the context of cancers, to restore an anti-tumor activity of macrophages and/or prevent the pro-tumoral activity of M2-type macrophages. Indeed, immune responses due to an excess of M2-type macrophage polarization also occur in infectious diseases, vaccination, trauma and chronic inflammatory diseases.

Macrophages are supposed to interact with stem cells or progenitor cells to control repair and remodeling functions. Cells from macrophages lineage present some neuroprotective effect. Mesenchymal cells (MSC) are targeted to promote tissue repair and immunoregulation. Injection of MSC was associated with a benefit for the recovery of functions of the spinal cord injury such as axonal preservation and reduced scare formation. The neuroprotective effect was attributed to a polarization shift from M2 to M1 macrophages by MSC (Nakajima et al., 2012), indicating that any molecules enabling this shift, such as an anti-SIRPa compound, could have a neuroprotective effect.

Macrophages are also involved in some iron deficiencies such as hemochromatosis, where the iron homeostasis is clearly impaired. *Polycythemia vera* or essential *polycythemia rubra* is a myeloproliferative disorder characterized by polycythemia (significant increase in the number of red blood cells) and an increase in the total cell volume. Red cells subsequently pass into the blood and could progress into a myeloproliferative syndrome. Patient treated with an anti-CD47 present anemia indicating that the blockade of CD47 is not as safe as expected; targeting SIRPa through an anti-SIRPa compound in the sense of the present invention could avoid this side effect.

The present invention thus pertains to the use of an anti-SIRPa compound, for modifying macrophage polarization to favor M1 pro-inflammatory macrophages in an individual suffering from a cancer, an infectious disease, a trauma, an auto-immune disease, a neurologic disease, a brain injury, a nerve injury, a polycythemia, a hemochromatosis or a chronic inflammatory disease, as well as in a context of vaccination of an individual.

According to a particular embodiment, the anti-SIRPa compound is used to treat an individual who has a cancer selected from the group consisting of lung cancers, ovary cancers, liver cancers, bladder cancers, brain cancers, breast cancers, colon cancers, thymomas, gliomas, melanomas and hematologic cancers.

In a particular embodiment, the anti-SIRPa compound is used in the treatment of any cancer with the exception of SIRPa-positive acute myeloid leukemia (AML). Indeed, the treatment of tumor cells involved in AML, which express SIRPa is thus directed toward the tumor cells, i.e. through a direct targeting of the tumor. This therapeutical strategy is thus different from the indirect approach proposed in the present invention. In a more particular embodiment, the anti-SIRPa compound is used in the treatment of any cancer with the exception of SIRPa-positive acute myeloid leukemia and SIRPa-positive non acute myeloid leukemia. In another particular embodiment, the anti-SIRPa compound is used in the treatment of any cancer with the exception of SIRPa-positive non-Hodgkin lymphoma or non-Hodgkin lymphoma. In a further particular embodiment, the anti-SIRPa compound is used in the treatment of any cancer with the exception of SIRPa-positive acute myeloid leukemia and/or SIRPa-positive non acute myeloid leukemia and/or non-Hodgkin lymphoma or hematologic cancers. In a further embodiment, the anti-SIRPa compound is used in the treatment of any cancer with the exception of i) SIRPa-positive acute myeloid leukemia or acute myeloid leukemia, and/or ii) SIRPa-positive non acute myeloid leukemia or non acute myeloid leukemia, and/or iii) SIRPa-positive non-Hodgkin lymphoma or non-Hodgkin lymphoma, or iv) SIRP-a positive hematologic cancers or hematologic cancers. In a further embodiment, the anti-SIRPa compound is used in the treatment of cancer with SIRPa-negative tumor cells, as described thereafter in the description.

In another particular embodiment, the anti-SIRPa compound is used in a monotherapy, in particular in the treatment of breast cancer, hepatocarcinoma or melanoma.

Modulation macrophage polarization is a very attractive approach to treat cancer especially in a combined therapy of cancer. Antonia et al. (Antonia et al., 2014) defined immuno-oncology combination that could be interesting for cancer treatment using checkpoint inhibitor approaches. Immuno-oncology is an evolving treatment modality that includes immunotherapies designed to harness the patient's own immune system.

In this context, an anti-SIRPa compound, in the sense of the present invention, can be combined with some other potential strategies for overcoming tumor immune evasion mechanisms with agents in clinical development or already on the market (see table 1 from (Antonia et al., 2014)):
1- Reversing the inhibition of adaptive immunity (blocking T-cell checkpoint pathways), for example by using an anti-CTLA4, an anti-PD1 or an PD-L1 molecule;
2- Switching on adaptive immunity (promoting T-cell costimulatory receptor signaling using agonist antibodies), for example by using an anti-CD137 molecule;
3- Improving the function of innate immune cells;
4- Activating the immune system (potentiating immune-cell effector function), for example through vaccine-based strategies.

Recently, Zitvogel et al. highlighted the importance of the intestinal microbiome for optimal therapeutic immunomodulation (Viaud et al., 2014; WO 2015/075688). In the frame of the present invention, an anti-SIRPa compound can also be combined with a microbiome-modulating strategy to improve the anti-cancer immune response.

Another aspect of the present invention is thus the use of an anti-SIRPa compound as above-defined, in combination with a second therapeutic agent, to treat an individual in need thereof, in particular a cancer patient. Such combinations can be used simultaneously separately or sequentially, particularly, in the treatment of cancer.

According to preferred embodiments of this aspect of the present invention, the second therapeutic agent is selected from the group consisting of chemotherapeutic agents, radiotherapy, surgery, immunotherapeutic agents, antibiotics and probiotics. In particular, the second therapeutic agent can advantageously be selected from the group consisting of therapeutic vaccines, immune checkpoint blockers such as, for example, anti-PDL1, anti-PD1 and anti-CTLA4 and immune checkpoint activators such as, for example, anti-CD137. As exemplified in the experimental part below, these combinations produce synergistic effects. In particular, on aspect of the invention consists in the use of an anti-SIRPa compound in combination with a second immune checkpoint modulator in the treatment of cancer selected among hepatocarcinoma or melanoma. In a preferred embodiment, the anti-SIRPa mAb is combined with an anti-CD137 mAb in the treatment of hepatocarcinoma. In another aspect of the invention, the anti-SIRPa mAb is combined with an anti-PD-L1 mAb in the treatment of melanoma.

Another aspect of the present invention is a method for *ex vivo* obtaining pro-inflammatory M1-type macrophages, comprising a step of incubating macrophages with an anti-SIRPa compound as described above. This method can be useful, for example, in cell therapy, especially for cancer patients. The present text also describes a method for treating a cancer patient, comprising a step of obtaining macrophages from said patient, followed by a step of modifying their polarization to favor M1-associated pro-inflammatory functions through incubation with an anti-SIRPa compound, and a step of re-administering the obtained pro-inflammatory cells to the patient. Of course, additional steps (such as expanding the cells, selecting the cells which exhibit a M1-type phenotype and/or counter-selecting those which exhibit a M2-type phenotype) can be introduced in such a method.

According to another of its aspects, the present invention pertains to a method for *in vivo* determining the efficacy of a treatment by an anti-SIRPa compound as defined above, comprising measuring the presence of pro-inflammatory M1-type macrophages and/or measuring the presence of M2-type macrophages in a sample from an individual treated by said compound. When performing the method, the presence of pro-inflammatory macrophages can be measured, for example, by measuring the levels of IL6, TNF-α and/or IL12p40 secreted by the macrophages present in the sample. The presence of M2-type macrophages can be measured, for example, by measuring the expression of CD206, CD11b, PD-L1 and/or CD200R on the surface of macrophages.

In order to avoid an unnecessary treatment with an anti-SIRPa compound, it is of major importance to correctly identify patients who are likely to benefit from such a treatment, *i.e.,* patients for whom this treatment will be efficient. Patients exhibiting high levels of M2-type macrophages, especially cancer patients having a high quantity of tumor-infiltrating M2-type macrophages, are those who are the most likely to respond to a treatment with an anti-SIRPa according to the present invention.

It is one aspect of the invention to be able to treat patients presenting a SIRPa-negative tumor, i.e. to propose the use of an anti-SIRPa compound, alone or in combination, to patients wherein the tumor cells do not express SIRPa. This therapeutic approach could not have been envisaged before, as prior art taught that the treatment of cancer relied on the inhibition of the SIRPa-CD47 interaction at tumor cell level (especially by inducing phagocytosis). The inventors of the present invention demonstrated that, on the contrary, anti-SIRPa mAb do not induce phagocytosis and act on a distinct pathway. They demonstrated that anti-SIPRa compounds target the M2-type macrophages and allow to repolarize this population of macrophages into M1-type macrophages. Thus, the proposed invention responds to a "new clinical situation" not described before. To go further in the difference between the claimed invention and the prior art teaching, the anti-SIRPa compound of the invention allows to treat cancer by indirect approach, targeting the innate immune system, in particular by inhibiting the inhibition of inflammation in tumor environment, and more specifically by inhibiting the anti-inflammatory M2-type macrophages.

Accordingly, an object of the invention consists in the use of an anti-SIRPa compound in the treatment of cancer, wherein the said compound is administered to a patient presenting a SIRPa-negative tumor. As used herein, a "SIRPa-negative tumor" corresponds to a tumor containing a SIRPa-negative cellular population. However, taking into account the heterogenous nature of a tumor, this term encompasses both a tumor consisting in SIRPa-negative cells and a tumor which may contain a mixed population of SIRPa-positive tumor cells and SIRPa-negative tumor cells. In a particular embodiment, the present invention also concerns the use of said compound for treating cancer wherein the tumor of the patient comprises a mixed population of tumor cells containing both SIRPa-positive and SIRPa-negative cells.

The present invention also provides a method for treating cancer comprising the administration of an anti-SIRPa compound able to inhibit the polarization of anti-inflammatory M2-type macrophage and/or to favor pro-inflammatory M1-type macrophage, to a patient in need thereof.

In a particular embodiment, the present invention also concerns a combination product comprising:
- at least one anti-SIRPa compound able to inhibit the polarization of anti-inflammatory M2-type macrophages and/or favors pro-inflammatory M1-type macrophage, in particular said anti-SIRPa compound being selected from the group of an antagonist peptide, an anti-SIRPa antibody, in particular an anti-SIRPa antagonist antibody, a nucleic acid encoding such compound, and a compound able to inhibit the expression of the SIRPa protein, in particular a siRNA; and
- a second therapeutic agent, in particular said second therapeutic agent being another immune checkpoint compound selected from the group consisting of anti-PDL1, anti-PD1, anti-CTLA4 and anti-CD137;
for use in the treatment of cancer. In a particular embodiment, the combinaison product is used to treat cancer, with the exception of SIRPa-positive acute myeloid leukemia.

The advantageous embodiments are as defined above.

The present invention hence also pertains to a method for *in vivo* determining if an individual is likely to be a good responder to a treatment by an anti-SIRPa compound in the sense of the present invention, comprising measuring the presence of M2-type macrophages in a sample from said individual, for example by measuring the expression of CD206, CD11b, PD-L1 and/or CD200R on the surface of macrophages present in the sample.

When performing the above methods, the sample used either to assess whether an individual is likely to be a good responder to a treatment with an anti-SIRPa compound or to determine the efficacy of such a treatment can be a blood sample, a tissue sample, a sample from a tumor or a sample of synovial liquid.

### EXAMPLES

Throughout the experimental part disclosed below, the terms used are in accordance with the scientific community working on macrophages (Murray et al., 2014).

The experimental results have been obtained with the materials and methods which follow:

### Human Blood Monocyte cell isolation

PBMC (peripheral blood Mononuclear cells) were isolated and purified using centrifugal counterflow elutriation (Clinical Transfer Facility CICBT0503, Dr. M. Gregoire, Nantes) by the protocol used and described by Coulais et al. (Coulais et al., 2012).

### Blocking antibodies

All the blocking molecules tested in the experiments were used at 10 µg/mL.

SIRPa mAbs: SE7C2 (Santa Cruz sc-23863) or clone p84 (Merck Millipore); Sirp α /β mAbs: SE5A5 (BioLegend BLE323802); anti-CD47 : B6H12 (eBioscience 14-0479-82) or CC2C6 (BioLegend BLE323102); CD47-Fc : SinoBiological 12283-H02H; anti-CD137 antibody (clone 3H3 produced in-house) and anti-PD-L1 antibody (10F-9G2 from BioXCell)

### In vitro M-CSF + (IFNg or IL4) Macrophage polarization assays (M1 or M2 type)

Conventional differentiation (Zajac et al., 2013) was performed by culturing monocytes in 24-well plates at 4.10⁵ cells/well in complete RPMI for 5 to 6 days in M-CSF (100µg/mL - R&D systems) to induce M0 macrophages. M2 polarisation is induced by rhIL-4 (20ng/mL-CellGenix) to generate M2 anti-inflammatory macrophages or by rhIFNg +/- LPS (20ng/mL - R&D systems ; 100ng/mL - Sigma-Aldrich, respectively) to generate M1 pro-inflammatory macrophages for 2 days, then cells were harvested and were analysed by Flow Cytometry using antibodies from BD Bioscience.

### In vitro CSF+GM-CSFMacrophage polarization assays (M1 and M2 types)

M1/M2 differentiation in the same well was realised by culturing monocytes in 24-well plates at 4.10⁵ cells/well in complete RPMI with GM-CSF (10ng/mL - CellGenix) for 3 days and then with GM-CSF and M-CSF (2ng/ml and 10ng/mL, respectively - R&D systems) for 3 supplementary days (Haegel et al., 2013) . Antibodies were added at day 0 and day 3. At day 6, cells were harvested and were analysed by Flow Cytometry using antibodies from BD Bioscience.

Secreted cytokines were titrated by ELISA (BD Bioscience and R&D systems).

### Macrophage phenotype by Flow Cytometry

In vitro mouse macrophages differentiation was performed by culturing cells from the bone marrow in 24-well plates at 0,5.10⁶ cells/mL in complete RPMI for 4 days supplemented by murine M-CSF (100µg/mL - Peprotech) to induce M0 macrophages. M2 polarization is induced by murine IL-4 (20ng/mL - Peprotech) to generate M2 anti-inflammatory macrophages for 24 hours. Analysis of phenotype changes was performed by flow cytometry staining using antibodies from BD Bioscience.

Expression markers of macrophages were analysed by flow cytometry using as the fluorochromes referred to in the table below:

| **Name** | **Fluorochrome** | **Reference** | **Isotype** | **Species** | **Providers** | **Dilution** |
|---|---|---|---|---|---|---|
| CD11b | Pacific blue | 558123 | IgG1 | Mouse | BD | 1/100 |
| CD197 (CCR7) | Pe-Cy7 | 557648 | IgG2a | | BD | |
| HLA-DR | APC-Cy7 | 335831 | IgG2a | Mouse | BD | |
| CD86 | PECy5 | 555659 | IgG1 | Mouse | BD | |
| CD200R | A647 | MCA2282A647 | IgG1 | Mouse | AbD Serotec | |
| CD206 | FITC | 551135 | IgG1 | Mouse | BD | |

### iNOS measurement by Multiplexed Nanoflares

iNOS expression was revealed by the SmartFlare^{™} technology (Prigodich et al., 2012). Briefly, monocytes or macrophages were collected and incubated with the iNOS probe (1nM final) 2h at 4°C, washed and analysed on a LSR II (BD).

### siRNA experiment

siRNA coding for endogenous SIRPa were transfected into macrophages (M0-macrophages pre-polarized by M-CSF). Three sequences of siRNA (ThermoFisher Scientific, ref: 112328, 112327 and 109944) were chosen and pooled to downregulate SIRPa expression. In a 24-well plate, 90 pmol of siRNA-SIRPa (3*30pmol of each siRNA-SIRPa) were diluted in 100µl Opti-MEM Medium without serum and mixed gently. Then 1µl/well of lipofectamine RNAiMAX (ThermoFisher Scientific, ref 13778-150) was mixed and incubated for 20 min. at room temperature. M2 were plated at 100 000 cells/ml in 500µl of complete growth medium with IL4 (20ng/ml) +/- 100µl of the siRNA-lipofectamine complexes. Null-siRNA was used as a control of transfection. Cells were incubated 48 hours at 37°C, 5%CO₂.

### Functional assay by Elisa

Cytokines and chemokines released in the supernatant were analyzed by Elisa using materials of BD Bioscience and R&D systems (references below), according to the manufacturer's instructions. Supernatants were diluted at 1/200.

| **Name** | **References** | **Species** | **Providers** |
|---|---|---|---|
| CCL17/TARC | Dy364 | Human | R&D systems |
| IL-6 | 555220 | | BD |
| IL12p40 | 55171 | | BD |
| CCL-2 | DY279 | | R&D systems |
| TNF-α | 555212 | | BD |

PK136 anti-NK1.1 (mouse mAb IgG2a) and SF1-1.1 anti-H2Kd (mouse mAb IgG1) were used as isotypic control antibodies.

### Evaluation of phagocytosis activity of human macrophage

To assert the ability of anti-SIRPa antibody to induce phagocytosis, Human M1 pro-inflammatory macrophages were generated as previously described and stained with a fluorescent dye. The CD47-expressing Raji cells were stained with another fluorescent dye and incubated with M1 stained macrophages during 2h at 37°C. Cells were fixed with paraformaldehyde and analysis of phagocytosis was evaluated by flow cytometry by gating on M1 fluorescent macrophages and analysis of target (Raji) cells fluorescence into M1 macrophages.

### In vivo mice hepatocarcinoma model

Eight-weeks-old C57Bl/6J male mice received 2.5 x 10⁶Hepa1.6 mouse hepatoma cells in 100 µL through the portal vein, as previously described (Gauttier et al., 2014). Four and eight days after tumour inoculation, mice were injected intraperitoneally with 100 µg of rat anti-4-1BB mAb (clone 3H3 produced in house), or with 300µg of anti-mouse SIRPa monoclonal antibody (clone P84 from Merck Millipore) or both antibodies or an irrelevant control antibody (clone 3G8) 3 times/week for 4 weeks or with 200 µg of the anti-PD-L1 mAb (clone 10F-9G2 from BioXCell) or received both antibodies (anti-Sirpa + anti-PDL1) for 4 weeks.

### In vivo mice melanoma model

Eight-weeks-old C57Bl/6J male mice received subcutaneous injection of 2 x 10⁶ B16-Ova mouse melanoma cells into the flank. Mice were treated i.p. from day 0 after tumor inoculation with either 300µg of an irrelevant control antibody (clone 3G8) or anti-mouse SIRPa monoclonal antibody (clone P84) 3 times per week or with 200 µg of the anti-PD-L1 mAb (clone 10F-9G2 from BioXCell) twice a week or received both antibodies (anti-Sirpa and anti-PD-L1 antibodies) for 4 weeks. Some animals were sacrificed at two weeks after tumor inoculation to characterized tumor leukocytes infiltrates by flow cytometry. The overall survival was analyzed.

### In vivo mice breast cancer model

Eight-weeks-old Balb/c femelle mice were injected with 0,25.106 4T1 (mammary carcinoma) cells in the mammary gland in 50µL. Mice were treated i.p. from day 4 after tumor injection with either 300µg of an irrelevant control antibody (clone 3G8) or the anti-mouse SIRPa blocking antibody (clone P84) three times a week and during four weeks. Mice were euthanized six weeks after tumor inoculation. Tumor measurement was performed every 2-3 days and the tumor volume was determined according the calcul : length * width * Pi/6 (mm3).

### Example 1: In vitro study of the macrophage polarization (M1 and M2) and blocking SIRPa pathway

### 1.1. Selective blockade of Sirp alpha prevents human macrophage polarization in type 2 (M2) but not in type 1 (M1)

Figure 1A shows that antibodies directed against SIRP molecules or CD47 do not prevent M1 macrophage polarization induced by GM-CSF + M-CSF because the expressions of the M1 cell surface markers (CD86 and CCR7) are not modified compared to control conditions. In contrast, the over-expression of CD206, CD200R, CD11b and PD-L1 (hallmarks of M2 macrophage phenotype) was significantly inhibited with selective anti-SIRP alpha mAb (Figure 2A and Figure 11) but not with control antibodies (anti-SIRPa/b or Sirpb), CD47-Fc recombinant protein or anti-CD47 mAbs (clones B6H12 and CC2C6). In particular, Figure 11 shows that blockade of SIRPa by a monoclonal antibody prevents the acquisition of the M2 macrophage phenotype induced by IL-4, indeed the expression of M2 markers (CD206, CD11b, and PD-L1) did not raise whereas it was observed in the isotype control condition. This prevention of anti-inflammatory status of macrophage was not observed when the cognate ligand of SIRPa (CD47) was blocked with a monoclonal antibody.

Measurement of cytokines and chemokines secretion showed that anti-SIRPa mAb prevented CCL-17 secretion (a hallmark chemokine of M2 secretion) while they increased secretion of pro-inflammatory cytokines (IL-6, IL12p40, TNF-α) and chemokine (CCL-2) secreted by M1 macrophages (Figure 1B and 2B). Anti-SIRPa thus seems to play a role on the prevention of M2 polarization and on the pro-inflammatory function of the macrophages. The selective inhibition of M2 macrophage polarization by only anti-SIRPa mAb was confirmed when monocytes were exclusively and terminally differentiated in M2 (not M1) macrophages with M-CSF + IL-4 protocol (Figure 5A.), while M1 polarization was not modified when monocytes were treated with M-CSF+IFN γ (data not shown). Again, only SIRPa mAb (not others anti-SIRP molecules mAb or anti-CD47 mAbs) prevented over-expression of surface markers typical of M2 (CD206 and CD200R), while in same time they increased IL-6 pro-inflammatory cytokine secretion (Figure 5 B). The role of SIRPa on M2 polarization was then studied at a transcriptional level with SIRPa inhibition, using a cocktail of siRNAs. The results, presented in Figure 6A, confirmed that inhibiting SIRPa inhibits M2 polarization of the cells (CD206 and CD200R expression).

Taken together, these results demonstrate that the inhibition of the M2-type macrophage can be obtained by inhibiting the SIPRa-CD47 interaction using anti-SIRPa antibody, but not using anti-CD47 antibody. Further, the inhibition of the M2 phenotype is observed both at phenotypic level (inhibition of the expression of M2-specific surface markers) and at functional level (inhibition of M2-specific cytokine secretion).

### 1.2. Selective blockade of SIRPa increases the secretion of pro-inflammatory factors, characteristic M1 macrophages

The inventors observed that selective anti-SIRPa mAb increases inflammatory cytokines secretion (IL6, IL12p40, CCL2 and TNFα) under M1+M2 polarization with GM-CSF + M-CSF (Figure1B). This property was confirmed in a conventional M1 (only) macrophage polarization assay induced by M-CSF + IFNg +/- LPS (Figure 3), showing an increase of IL6 and IL12p40 in the supernatant. Maximal/terminal M1 polarization is considered achieved using M-CSF + IFNg + LPS, which results in all hallmarks of M1 macrophage, in particular iNOS expression. Figure 4 shows the expression of iNOS in M1 polarized cells treated or not with different blocking antibodies. As shown in this figure, blocking SIRPa with an antibody increased the expression of iNOS. However, anti-SIRPβ or anti-CD47 mAb did not induce any modification in the iNOS expression profile. These results confirmed that blocking SIRPa modifies macrophage function in a pro-inflammatory state such as M1 type. However, the M1 surface markers tested were not modified compared to the controls.

### 1.3. Selective blockade of SIRPa repolarizes human M2 macrophages in inflammatory cells

The phenotype and function of polarized M1/M2 macrophages has been, to some extent, reversed *in vitro* or *in vivo* due to the plasticity of these cells (Sica and Mantovani, 2012). The inventors addressed the question of the repolarization of the M2 type into a pro-inflammatory M1 type by blocking SIRPa. To do so, monocytes were cultivated with M-CSF + IL-4 inducing M2 terminal polarization. Then M2 cells were treated with different blocking antibodies. Results presented Figure 7 show that the anti-SIRPa mAb is able to repolarize M2 macrophages into M1 pro-inflammatory macrophages, since IL6 and TNF-α cytokines were induced (hallmark cytokines of M1 macrophages). This effect was not observed when cells were treated with an anti-Sirpβ nor with CD47-Fc nor anti-CD47 antibodies. As explained above, cell surface markers were not modified. Similarly, results of Figure 12 show that two different anti-SIRPa mAb allow to induce the polarization of macrophage in favor of M2-type macrophages, both at phenotypic level with expression of surface markers CD200R and CD80 (A) and at functional level with secretion of IL-6 (B). Further, results of Figure 14 show that the selective blockade of SIRPa but not CD47 prevents M2 polarization (B) and did not affect M1 polarization (A). Indeed, neither the addition of the specific monoclonal antibody of rat SIRPa, nor the anti-CD47 monoclonal antibody during polarization of pro-inflammatory M1-type macrophages affects the secretion of pro-inflammatory cytokines (IL-6 & TNF-a). On the contrary, the specific blockade of SIRPa (but not with anti-CD47) during M2 polarization of rat macrophages switches their cytokine profile towards an inflammatory profile (like M1 macrophages) with the secretion of IL-6 and TNF-α.

These results showed that SIRPa is important for the M2 polarization of the monocyte and blocking this pathway is a good opportunity to produce macrophages with pro-inflammatory profile to treat pathologies in need thereof such as cancer or for infectious diseases, in which macrophages are blocked in an M2 state.

Taken together, all these results demonstrate that all anti-SIRPa antibodies tested in these experiments (whether directed against the human, mouse or rat SIRPa) are able to modulate the macrophage polarization by inhibiting the M2-type macrophage phenotype. On the contrary, anti-CD47 antibodies has no effect on macrophage polarization.

### 1.4 Anti-SIRPa antibody does not increase the human macrophage phagocytosis on tumor cells expressing CD47

The ability of an anti-SIRPa antibody (clone SE7C2) to induce phagocytosis was assayed on human macrophage. Figure 13 shows that two different anti-CD47 mAbs increase the phagocytosis of CD47+ tumor cells (Raji) by M1 macrophages as described in literature. In contrast, the selective blockade of SIRPa by the anti-SIRPa mAb or a recombinant CD47-Fc fusion protein, do not affect increase phagocytic activity of M1 macrophages. These results suggest that phagocytosis of CD47+ tumor cells are not induce by SIRPa/CD47 interaction but rather by an ADCP (Antibody-dependent cellular phagocytosis) mechanism. Further, these results confirm that anti-SIRPa compounds cannot induce phagocytosis of tumor cells.

### Example 2: In vivo study of the effects of SIRPa blockade

### 2.1. Effect of the SIRPa blockade in an in vivo model of hepatocarcinoma

Figure 8 represents the overall survival rate of animals inoculated with hepatocarcinoma and treated with an anti-CD137, an anti-SIRPa or both during 4 weeks. 20% of the animals treated with anti-Sirpa monotherapy survived more than 25 days and 25% of animals treated with anti-CD137 monotherapy survived more than 30 days. However, 100% of the animals receiving the combo anti-Sirp+anti-CD137 were still alive after 80 days, compared to the other conditions, showing a synergistic effect of the 2 molecules.

Figure 9 represents the overall survival rate of animals inoculated with hepatocarcinoma and treated with an anti-PD-L1, an anti-SIRPa or both during 4 weeks. The results showed a very interesting surviving rate when animals were treated with both molecules, compared to each molecule alone (20% of alived animals after 20 days with anti-sirpa compare to 12% of alived animals with anti-PD-L1). This result indicates a synergistic effect of the anti-SIRPa antibody with the anti-PD-L1 antibody in a cancer model.

### 2.2. Effect of the SIRPa blockade in an in vivo model of melanoma

Figure 10 A represents the overall survival rate of animals inoculated with melanoma cells and treated with an anti-PD-L1, an anti-SIRPa or both during 4 weeks. Compared to the treatment with each molecule alone, the combination showed a better efficacy. Figure 10 B shows the macrophage infiltrate in animals treated with anti-SIRPa, confirming an increase in macrophage number into the tumor.

The *in vivo* experiments on 2 different cancer models showed that SIRPa is an interesting target for cancer treatment, especially when combined with other immunotherapies, and suggest that Sirp is a new checkpoint inhibitor that is important to block in the aim to restore a pro-inflammatory tumor environment.

### 2.3. Effect of the SIRPa blockade in an in vivo model of mammary cancer.

Figure 15 shows that monotherapy with anti-SIRPa mAb (clone P84) inhibit tumor development in a syngeneic and orthotopic triple-negative breast model in mice. From 2 weeks post-tumor inoculation until the end of the experiment, anti-SIRPa treated mice have a significant reduction in tumor volume.

The following numbered embodiments constitute preferred embodiments of the invention.
1. An anti-SIRPa compound able to inhibit the polarization of anti-inflammatory M2-type macrophages and/or favors pro-inflammatory M1-type macrophage, for use in the treatment of cancer, with the exception of SIRPa-positive acute myeloid leukemia.
2. The compound of embodiment 1 for use according to embodiment 1, wherein said compound is selected from the group consisting of an anti-SIRPa antibody, in particular an anti-SIRPa antagonist antibody, a nucleic acid encoding such compound, and a compound able to inhibit the expression of the SIRPa protein, in particular a siRNA.
3. The compound of embodiment 1 or 2 for use according to embodiment 1 or 2, wherein said cancer is selected from the group consisting of lung cancers, ovary cancers, liver cancers, bladder cancers, brain cancers, breast cancers, colon cancers, thymomas, gliomas, melanomas, leukemia and myeloma.
4. The compound of any one of embodiments 1 to 3 for use according to any one of embodiments 1 to 3, wherein said compound is administered to a patient presenting a SIRPa-negative tumor.
5. The compound of any of embodiments 1 to 4 for the use according to any of embodiments 1 to 4, wherein said compound is combined to a second therapeutic agent.
6. The compound of embodiment 5, for use according to embodiment 5, wherein said second therapeutic agent is selected from the group consisting of chemotherapeutic agents, radiotherapy, surgery, immunotherapeutic agents, antibiotics and probiotics.
7. The compound of embodiment 6, for use according to embodiment 6, wherein said second therapeutic agent is an immunotherapeutic agent selected from the group consisting of therapeutic vaccines and immune checkpoint blockers or activators.
8. The compound of embodiment 7, for use according to embodiment 7, wherein said second therapeutic agent is an immune checkpoint blocker or activator selected from the group consisting of anti-PDL1, anti-PD1, anti-CTLA4 and anti-CD137.
9. A method for *ex vivo* obtaining pro-inflammatory M1-type macrophages, comprising a step of incubating macrophages with an anti-SIRPa compound able to inhibit the polarization of anti-inflammatory M2-type macrophages and/or favors pro-inflammatory M1-type macrophage.
10. The method of embodiment 9, wherein said compound is selected from the group consisting of an anti-SIRPa antibody, in particular an anti-SIRPa antagonist antibody, a nucleic acid encoding such compound, and a compound able to inhibit the expression of the SIRPa protein, in particular a siRNA.

### REFERENCES

Adams, S., van der Laan, L.J., Vernon-Wilson, E., Renardel de Lavalette, C., Döpp, E.A., Dijkstra, C.D., Simmons, D.L., and van den Berg, T.K. (1998). Signal-regulatory protein is selectively expressed by myeloid and neuronal cells. J. Immunol. Baltim. Md 1950 161, 1853-1859.
Antonia, S.J., Larkin, J., and Ascierto, P.A. (2014). Immuno-oncology combinations: a review of clinical experience and future prospects. Clin. Cancer Res. Off. J. Am. Assoc. Cancer Res. 20, 6258-6268.
Barclay, A.N., and Van den Berg, T.K. (2014a). The interaction between signal regulatory protein alpha (SIRPa) and CD47: structure, function, and therapeutic target. Annu. Rev. Immunol. 32, 25-50.
Barclay, A.N., and Van den Berg, T.K. (2014b). The interaction between signal regulatory protein alpha (SIRPa) and CD47: structure, function, and therapeutic target. Annu. Rev. Immunol. 32, 25-50.
Chao, M.P., Tang, C., Pachynski, R.K., Chin, R., Majeti, R., and Weissman, I.L. (2011). Extranodal dissemination of non-Hodgkin lymphoma requires CD47 and is inhibited by anti-CD47 antibody therapy. Blood 118, 4890-4901.
Cornelissen, R., Heuvers, M.E., Maat, A.P., Hendriks, R.W., Hoogsteden, H.C., Aerts, J.G.J.V., Hegmans, J.P.J.J., Cornelissen, R., Heuvers, M.E., Maat, A.P., et al. (2012). New Roads Open Up for Implementing Immunotherapy in Mesothelioma, New Roads Open Up for Implementing Immunotherapy in Mesothelioma. J. Immunol. Res. J. Immunol. Res. 2012, 2012, e927240.
Coulais, D., Panterne, C., Fonteneau, J.-F., and Grégoire, M. (2012). Purification of circulating plasmacytoid dendritic cells using counterflow centrifugal elutriation and immunomagnetic beads. Cytotherapy 14, 887-896.
DeNardo, D.G., Andreu, P., and Coussens, L.M. (2010). Interactions between lymphocytes and myeloid cells regulate pro- versus anti-tumor immunity. Cancer Metastasis Rev. 29, 309-316.
Edris, B., Weiskopf, K., Volkmer, A.K., Volkmer, J.-P., Willingham, S.B., Contreras-Trujillo, H., Liu, J., Majeti, R., West, R.B., Fletcher, J.A., et al. (2012). Antibody therapy targeting the CD47 protein is effective in a model of aggressive metastatic leiomyosarcoma. Proc. Natl. Acad. Sci. U. S. A. 109, 6656-6661.
Gabrilovich, D.I., Ostrand-Rosenberg, S., and Bronte, V. (2012). Coordinated regulation of myeloid cells by tumours. Nat. Rev. Immunol. 12, 253-268.
Haegel, H., Thioudellet, C., Hallet, R., Geist, M., Menguy, T., Le Pogam, F., Marchand, J.-B., Toh, M.-L., Duong, V., Calcei, A., et al. (2013). A unique anti-CD115 monoclonal antibody which inhibits osteolysis and skews human monocyte differentiation from M2-polarized macrophages toward dendritic cells. mAbs 5, 736-747.
Hanahan, D., and Coussens, L.M. (2012). Accessories to the Crime: Functions of Cells Recruited to the Tumor Microenvironment. Cancer Cell 21, 309-322.
Janssen, W.J., McPhillips, K.A., Dickinson, M.G., Linderman, D.J., Morimoto, K., Xiao, Y.Q., Oldham, K.M., Vandivier, R.W., Henson, P.M., and Gardai, S.J. (2008). Surfactant proteins A and D suppress alveolar macrophage phagocytosis via interaction with SIRP alpha. Am. J. Respir. Crit. Care Med. 178, 158-167.
Majeti, R., Chao, M.P., Alizadeh, A.A., Pang, W.W., Jaiswal, S., Gibbs, K.D.J., van Rooijen, N., and Weissman, I.L. (2009). CD47 is an adverse prognostic factor and therapeutic antibody target on human acute myeloid leukemia stem cells. Cell 138, 286-299.
Mantovani, A., Biswas, S.K., Galdiero, M.R., Sica, A., and Locati, M. (2013). Macrophage plasticity and polarization in tissue repair and remodelling. J. Pathol. 229, 176-185.
McIlroy, D., Barteau, B., Cany, J., Richard, P., Gourden, C., Conchon, S., and Pitard, B. (2009). DNA/amphiphilic block copolymer nanospheres promote low-dose DNA vaccination. Mol. Ther. J. Am. Soc. Gene Ther. 17, 1473-1481.
Milling, S., Yrlid, U., Cerovic, V., and MacPherson, G. (2010). Subsets of migrating intestinal dendritic cells. Immunol. Rev. 234, 259-267.
Mittal, D., Gubin, M.M., Schreiber, R.D., and Smyth, M.J. (2014). New insights into cancer immunoediting and its three component phases--elimination, equilibrium and escape. Curr. Opin. Immunol. 27, 16-25.
Murray, P.J., Allen, J.E., Biswas, S.K., Fisher, E.A., Gilroy, D.W., Goerdt, S., Gordon, S., Hamilton, J.A., Ivashkiv, L.B., Lawrence, T., et al. (2014). Macrophage Activation and Polarization: Nomenclature and Experimental Guidelines. Immunity 41, 14-20.
Nakajima, H., Uchida, K., Guerrero, A.R., Watanabe, S., Sugita, D., Takeura, N., Yoshida, A., Long, G., Wright, K.T., Johnson, W.E.B., et al. (2012). Transplantation of mesenchymal stem cells promotes an alternative pathway of macrophage activation and functional recovery after spinal cord injury. J. Neurotrauma 29, 1614-1625.
Oldenborg, P.A., Zheleznyak, A., Fang, Y.F., Lagenaur, C.F., Gresham, H.D., and Lindberg, F.P. (2000). Role of CD47 as a marker of self on red blood cells. Science 288, 2051-2054.
Prigodich, A.E., Randeria, P.S., Briley, W.E., Kim, N.J., Daniel, W.L., Giljohann, D.A., and Mirkin, C.A. (2012). Multiplexed nanoflares: mRNA detection in live cells. Anal. Chem. 84, 2062-2066.
Qian, B.-Z., and Pollard, J.W. (2010). Macrophage Diversity Enhances Tumor Progression and Metastasis. Cell 141, 39-51.
Seiffert, M., Cant, C., Chen, Z., Rappold, I., Brugger, W., Kanz, L., Brown, E.J., Ullrich, A., and Bühring, H.J. (1999). Human signal-regulatory protein is expressed on normal, but not on subsets of leukemic myeloid cells and mediates cellular adhesion involving its counterreceptor CD47. Blood 94, 3633-3643.
Sica, A., and Mantovani, A. (2012). Macrophage plasticity and polarization: in vivo veritas. J. Clin. Invest. 122, 787-795.
Stein, M., Keshav, S., Harris, N., and Gordon, S. (1992). Interleukin 4 potently enhances murine macrophage mannose receptor activity: a marker of alternative immunologic macrophage activation. J. Exp. Med. 176, 287-292.
Teng, M.W.L., Galon, J., Fridman, W.-H., and Smyth, M.J. (2015). From mice to humans: developments in cancer immunoediting. J. Clin. Invest. 125, 3338-3346.
Ugel, S., Sanctis, F.D., Mandruzzato, S., and Bronte, V. (2015). Tumor-induced myeloid deviation: when myeloid-derived suppressor cells meet tumor-associated macrophages. J. Clin. Invest. 125, 3365-3376.
Uluçkan, O., Becker, S.N., Deng, H., Zou, W., Prior, J.L., Piwnica-Worms, D., Frazier, W.A., and Weilbaecher, K.N. (2009). CD47 regulates bone mass and tumor metastasis to bone. Cancer Res. 69, 3196-3204.
Viaud, S et al. (2014) Harnessing the intestinal microbiome for optimal therapeutic immunomodulation. Cancer Res. 2014 Aug 15;74(16):4217-21.
Wang, Y., Xu, Z., Guo, S., Zhang, L., Sharma, A., Robertson, G.P., and Huang, L. (2013). Intravenous delivery of siRNA targeting CD47 effectively inhibits melanoma tumor growth and lung metastasis. Mol. Ther. J. Am. Soc. Gene Ther. 21, 1919-1929.
Weiskopf, K., Ring, A.M., Ho, C.C.M., Volkmer, J.-P., Levin, A.M., Volkmer, A.K., Ozkan, E., Fernhoff, N.B., van de Rijn, M., Weissman, I.L., et al. (2013). Engineered SIRPa variants as immunotherapeutic adjuvants to anticancer antibodies. Science 341, 88-91.
Willingham, S.B., Volkmer, J.-P., Gentles, A.J., Sahoo, D., Dalerba, P., Mitra, S.S., Wang, J., Contreras-Trujillo, H., Martin, R., Cohen, J.D., et al. (2012). The CD47-signal regulatory protein alpha (SIRPa) interaction is a therapeutic target for human solid tumors. Proc. Natl. Acad. Sci. U. S. A. 109, 6662-6667.
Wynn, T.A., Chawla, A., and Pollard, J.W. (2013). Macrophage biology in development, homeostasis and disease. Nature 496, 445-455.
Zhao, X.W., van Beek, E.M., Schomagel, K., Van der Maaden, H., Van Houdt, M., Otten, M.A., Finetti, P., Van Egmond, M., Matozaki, T., Kraal, G., et al. (2011). CD47-signal regulatory protein-α (SIRPa) interactions form a barrier for antibody-mediated tumor cell destruction. Proc. Natl. Acad. Sci. U. S. A. 108, 18342-18347.

## Claims

1. An anti-SIRPa compound selected from the group consisting of an antibody or fragment(s) thereof, wherein the anti-SIRPa compound binds specifically to the extracellular domain of SIRPa and blocks SIRPa pathway, combined with
a second therapeutic agent selected from the group consisting of a blocking anti-CTLA-4 antibody and an agonistic anti-CD137 antibody,
for use in the treatment of a solid cancer.

2. The anti-SIRPa compound of claim 1, for use according to claim 1, wherein the cancer is a lung cancer, an ovary cancer, a liver cancer, a bladder cancer, a brain cancer, a breast cancer, a colon cancer, a thymoma, a glioma, or a melanoma.

3. The anti-SIRPa compound of claim 1 or 2, for use according to claim 1 or 2, wherein said cancer is selected from the group consisting of melanoma and colon cancer.
